# EUROPEAN PATENT SPECIFICATION

(11) **EP 2 619 333 B1**
(45) Date of publication and mention of the grant of the patent: **21.06.2017**
(21) Application number: 11827648.4
(22) Date of filing: 23.09.2011
(51) Int. Cl.: C12Q 1/68

(54) **NATIVE-EXTENSION PARALLEL SEQUENCING**
PARALLELSEQUENZIERUNG MIT NATIVER VERLÄNGERUNG
SÉQUENÇAGE PARALLÈLE D'EXTENSION NATIVE

(30) Priority: 23.09.2010 US 385947 P; 23.09.2010 US 385562 P
(43) Date of publication of application: 31.07.2013
(73) Proprietor: Centrillion Technology Holdings Corporation, Grand Cayman KY1-1104 (KY)
(72) Inventor: ZHOU, Wei, Los Altos, CA 94024 (US); MEI, Rui, Santa Clara, CA 95051 (US); LUCAS, Julian, Davis, CA 95618 (US); LIAO, Guochun, Belmont, CA 94002 (US)
(74) Representative: Roques, Sarah Elizabeth
(86) International application number: PCT/US2011/053079
(87) International publication number: WO 2012/040624

(56) References cited:
- WO-A1-92/10587
- GB-A- 2 398 383
- US-A1- 2003 064 376
- US-A1- 2005 186 576
- US-A1- 2006 275 782
- US-A1- 2009 088 327
- US-A1- 2010 029 498
- US-A1- 2010 143 900
- SEO ET AL.: 'Four-color DNA sequencing by synthesis on a chip using photocleavable fluorescent nucleotides.' PROC. NATL. ACAD. SCI. USA. vol. 102, no. 17, 26 April 2005, pages 5926 - 5931, XP002353000

## Description

### BACKGROUND OF THE INVENTION

Cost effective and fast sequencing is needed for microbial detection and identification, as well as practical identification of individuals, e.g., for paternity testing and in forensic science (Reynolds et al., Anal. Chem., 63:2-15 (1991)), for organ-transplant donor-recipient matching (Buyse et al., Tissue Antigens, 41:1-14 (1993) and Gyllensten et al., PCR Meth. Appl, 1:91-98 (1991)), for genetic disease diagnosis, prognosis, and pre-natal counseling (Chamberlain et al., Nucleic Acids Res., 16:11141-11156 (1988) and L. C. Tsui, Human Mutat., 1:197-203 (1992)), and the study of drug metabolism and oncogenic mutations (Hollstein et al., Science, 253:49-53 (1991)). In addition, the cost-effectiveness of infectious disease diagnosis by nucleic acid analysis varies directly with the multiplex scale in panel testing. Many of these applications depend on the discrimination of single-base differences at a multiplicity of sometimes closely spaced loci.

A variety of DNA hybridization techniques are available for detecting the presence of one or more selected polynucleotide sequences in a sample containing a large number of sequence regions. In a simple method, which relies on fragment capture and labeling, a fragment containing a selected sequence is captured by hybridization to an immobilized probe. The captured fragment can be labeled by hybridization to a second probe which contains a detectable reporter moiety.

Another widely used method is Southern blotting. In this method, a mixture of DNA fragments in a sample is fractionated by gel electrophoresis, and then fixed on a nitrocellulose filter. By reacting the filter with one or more labeled probes under hybridization conditions, the presence of bands containing the probe sequences can be identified. The method is especially useful for identifying fragments in a restriction-enzyme DNA digest which contains a given probe sequence and for analyzing restriction-fragment length polymorphisms ("RFLPs").

Another approach to detecting the presence of a given sequence or sequences in a polynucleotide sample involves selective amplification of the sequence(s) by polymerase chain reaction. U.S. Pat. No. 4,683,202 to Mullis, et al. and R. K. Saiki, et al., Science 230:1350 (1985). In this method, primers complementary to opposite end portions of the selected sequence(s) are used to promote, in conjunction with thermal cycling, successive rounds of primer-initiated replication. The amplified sequence(s) may be readily identified by a variety of techniques. This approach is particularly useful for detecting the presence of low-copy sequences in a polynucleotide-containing sample, e.g., for detecting pathogen sequences in a body-fluid sample.

More recently, methods of identifying known target sequences by probe ligation methods have been reported. U.S. Pat. No. 4,883,750 to N. M. Whiteley, et al., D. Y. Wu, et al., Genomics 4:560 (1989), U. Landegren, et al., Science 241:1077 (1988), and E. Winn-Deen, et al., Clin. Chem. 37:1522 (1991). In one approach, known as oligonucleotide ligation assay ("OLA"), two probes or probe elements which span a target region of interest are hybridized to the target region. Where the probe elements basepair with adjacent target bases, the confronting ends of the probe elements can be joined by ligation, e.g., by treatment with ligase. The ligated probe element is then assayed, evidencing the presence of the target sequence.

In a modification of this approach, the ligated probe elements act as a template for a pair of complementary probe elements. With continued cycles of denaturation, hybridization, and ligation in the presence of pairs of probe elements, the target sequence is amplified linearly, allowing very small amounts of target sequence to be detected and/or amplified. This approach is referred to as ligase detection reaction. When two complementary pairs of probe elements are utilized, the process is referred to as the ligase chain reaction which achieves exponential amplification of target sequences. F. Barany, Proc. Nat'l Acad. Sci. USA, 88:189-93 (1991) andF. Barany, PCR Methods and Applications, 1:5-16 (1991).

Another scheme for multiplex detection of nucleic acid sequence differences is disclosed in U.S. Pat. No. 5,470,705 to Grossman et al. where sequence-specific probes, having a detectable label and a distinctive ratio of charge/translational frictional drag, can be hybridized to a target and ligated together. This technique was used in Grossman, et al., Nucl. Acids Res. 22(21):4527-34 (1994) for the large scale multiplex analysis of the cystic fibrosis transmembrane regulator gene. Jou, et al., Human Mutation 5:86-93 (1995) relates to the use of a so called "gap ligase chain reaction" process to amplify simultaneously selected regions of multiple exons with the amplified products being read on an immunochromatographic strip having antibodies specific to the different haptens on the probes for each exon.

Solid-phase hybridization assays require multiple liquid-handling steps, and some incubation and wash temperatures must be carefully controlled to keep the stringency needed for single-nucleotide mismatch discrimination. Multiplexing of this approach has proven difficult as optimal hybridization conditions vary greatly among probe sequences.

Ligation of allele-specific probes generally has used solid-phase capture (U. Landegren et al., Science, 241:1077-1080 (1988); Nickerson et al., Proc. Natl. Acad. Sci. USA, 87:8923-8927 (1990)) or size-dependent separation (D. Y. Wu, et al., Genomics, 4:560-569 (1989) and F. Barany, Proc. Natl. Acad. Sci, 88:189-193 (1991)) to resolve the allelic signals, the latter method being limited in multiplex scale by the narrow size range of ligation probes. Further, in a multiplex format, the ligase detection reaction alone cannot make enough product to detect and quantify small amounts of target sequences. The gap ligase chain reaction process requires an additional step--polymerase extension. The use of probes with distinctive ratios of charge/translational frictional drag for a more complex multiplex will either require longer electrophoresis times or the use of an alternate form of detection.

There is a great need for rapid, high-throughput, and low cost sequencing technology, especially for markets such as point-of-care the field detection of pathogens. The present invention permits sequencing of large amount of genome using simple chemistry and low cost equipments that lead to significant cost reduction and increase in speed.

### SUMMARY OF THE INVENTION

The present invention provides a method of sequencing a target nucleic acid, comprising:
a) providing a plurality of substrates, wherein said plurality of substrates is n,
   each substrate of said plurality of substrates is designed as a different substrate (i), wherein said (i) is an integer from 1 to n, wherein each different substrate of said plurality of substrates comprises a capture site comprising a capture probe, and wherein said capture probe comprises a sequence that is complementary to a portion of said target nucleic acid, wherein said target nucleic acid comprises a fragment of a source nucleic acid;
b) forming a hybridization complex on each of said different substrates (i), wherein said hybridization complex comprises said capture probe and said target nucleic acid, wherein said capture probe is hybridized to said portion of said target nucleic acid;
c) extending said capture probe in said hybridization complex on each of said different substrates (i) by repeating i-1 times the step of: contacting said hybridization complex on each of said different substrates (i) sequentially with 1) one of native dATP, dCTP, dGTP and dTTP; or 2) a mixture of two or three native dNTPs followed by one or more rounds of two or more different native dNTPs until all four native dNTPs are added at least once, in the presence of a polymerase, thereby extending said capture probe in said hybridization complex by one or more bases using said target nucleic acid in said hybridization complex as template, wherein said extending said capture probe in said hybridization complex occurs in a separate extension reaction for each of said different substrates (i);
d) further extending said capture probe in said hybridization complex on each of said different substrate (i) sequentially with one of labeled dATP, dCTP, dGTP and dTTP in the presence of a polymerase;
e) detecting incorporation of said labeled dATP, dCTP, dGTP and dTTP in said hybridization complex on each of said different substrates (i), thereby obtaining a sequence read from said hybridization complex on each of said different substrates (i); and
f) determining a nucleotide sequence of said target nucleic acid by assembling said sequence reads, wherein said assembling is performed on a computer.

In one aspect, the present disclosure provides a method for determining the sequence of a target nucleic acid molecule, comprising: (a) providing a plurality of substrates, wherein the total number of substrates is n, each substrate is designed as substrate (i) and i is an integer from 1 to n, wherein each of said substrates comprises a capture site comprising a capture probe, and wherein each of said capture probe comprises a sequence that is complementary to a target nucleic molecule; (b) forming a plurality of hybridization complexes on said substrate, each complex comprises: said capture probe and a copy of said target nucleic acid molecule; (c) extending said capture probes on each said substrate (i) by repeating i-1 times the step of: contacting said hybridization complex on said substrate (i) sequentially with dATP, dCTP, dGTP and dTTP in the presence of a polymerase, thereby extending said capture probes by one or more bases using said target nucleic acid molecules as templates; (d) contacting said each hybridization complex on each said substrate (i), in the presence of a polymerase, sequentially with one of labeled dATP, dCTP, dGTP and dTTP, a mixture of labeled dATP, dCTP, dGTP and dTTP, a mixture of labeled ddATP, ddCTP, ddGTP and ddTTP, or a mixture of labeled ddATP, ddCTP, ddGTP, ddTTP and small amount (<10% or <20%) of native dATP, dCTP, dGTP, and dTTP; (e) detecting the incorporation of said of labeled dATP, dCTP, dGTP and dTTP to each of said capture probe on each of said substrate (i) to obtain a sequence read from each substrate (i); and (f) determining the sequence of said target nucleic acid molecule by assembling said sequence reads.

Also disclosed is a method for determining the sequence of a target nucleic acid molecule, comprising: (a) providing a plurality of substrates, wherein the total number of substrates is n, each substrate is designed as substrate (i) and i is an integer from 1 to n, wherein each of said substrates comprises a capture site comprising a capture probe, and wherein each of said capture probe comprises a sequence that is complementary to a target nucleic molecule; (b) forming a plurality of hybridization complexes on said substrate, each complex comprises: said capture probe and a copy of said target nucleic acid molecule; (c) extending said capture probes on each said substrate (i) by repeating i-1 times the step of: contacting said hybridization complex on said substrate (i) sequentially with one of dATP, dCTP, dGTP and dTTP, or a mixture of two or three of dATP, dCTP, dGTP and dTTP provided each of the four dNTPs is added at least once, in the presence of a polymerase, thereby extending said capture probes by one or more bases using said target nucleic acid molecules as templates; (d) contacting said each hybridization complex on each said substrate (i), in the presence of a polymerase, sequentially with one of labeled dATP, dCTP, dGTP and dTTP, a mixture of labeled dATP, dCTP, dGTP and dTTP, a mixture of labeled ddATP, ddCTP, ddGTP and ddTTP, or a mixture of labeled ddATP, ddCTP, ddGTP, ddTTP and small amount (<10% or <20%) of native dATP, dCTP, dGTP, and dTTP; (e) detecting the incorporation of said of labeled dATP, dCTP, dGTP and dTTP to each of said capture probe on each of said substrate (i) to obtain a sequence read from each substrate (i); and (f) determining the sequence of said target nucleic acid molecule by assembling said sequence reads.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

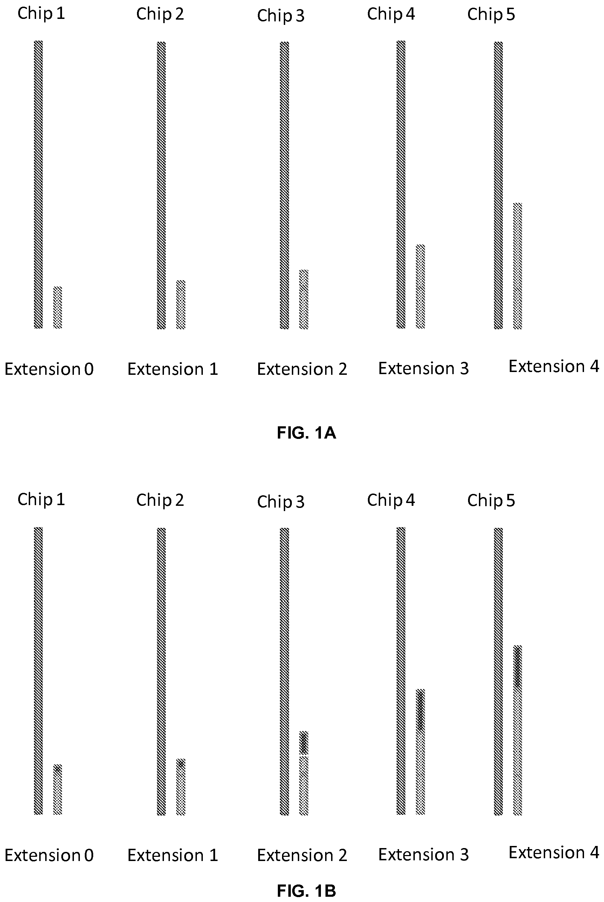

**FIGs. 1A and 1B** depict an exemplary embodiment of the invention. **FIG. 1A****:** Five different chips have identical capture probes attached and the probes hybridize to the target molecules. Each chip undergoes different number of extension cycles (here 0, 1, 2, 3, 4, and 5, respectively). For each extension cycle four different nucleotides (A, C, G, T) are added sequentially, one at a time. **FIG. 1B****:** The detection of four bases extension.

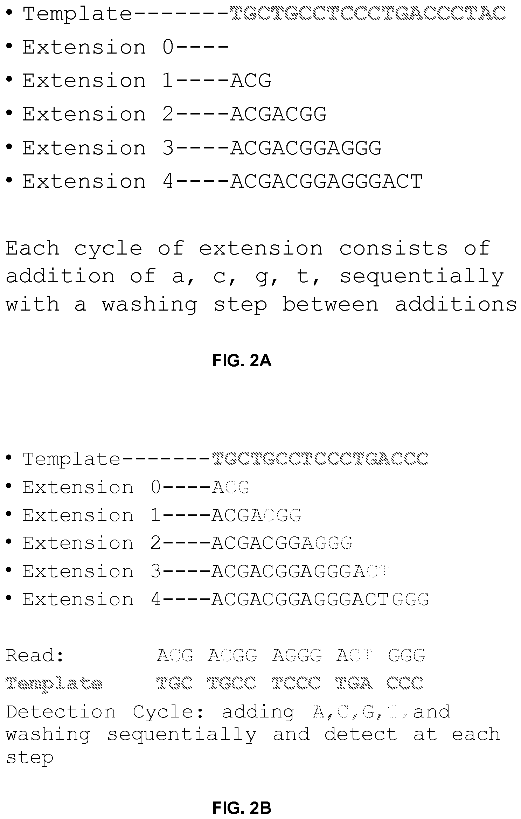

**FIGs. 2A-2B** depict the extension and detection of an exemplary target molecule. eukaryotic algae nuclear genetic engineering. **2A:** Extension. **2B:** Detection and labeled nucleotides.

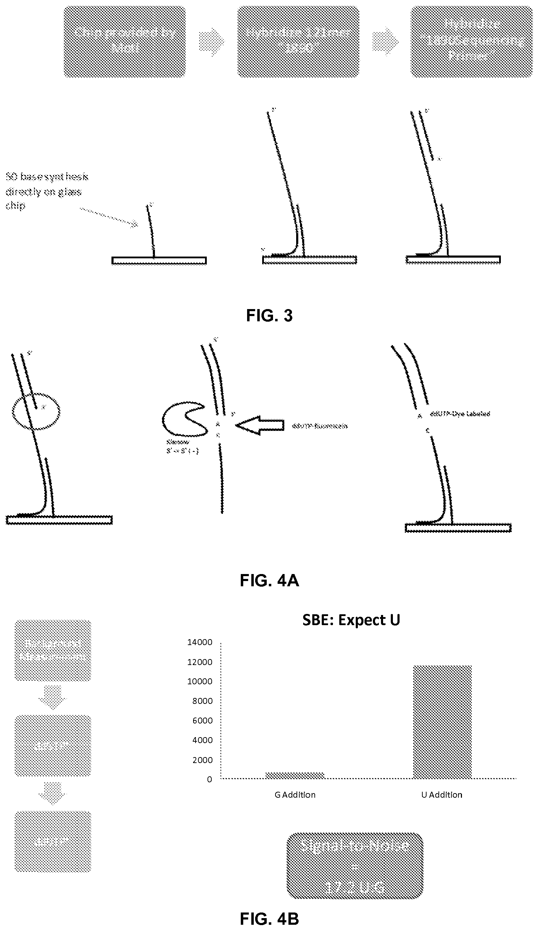

**FIG. 3A** depicts one exemplary embodiment of the invention. A capture probe (50 bases long) is synthesized directly on a glass chip and hybridizes to a 121 mer target nucleic acid molecule ("1890") and an 1890 sequencing primer is used for the sequencing reaction.
**FIG. 4A** depicts an exemplary embodiment of the invention, single-base extension (SBE). **FIG. 4B** depicts the experimental results of single-base extension. The signal-to-noise ratio between the labeled (U) and unlabeled (G) is 17.2.

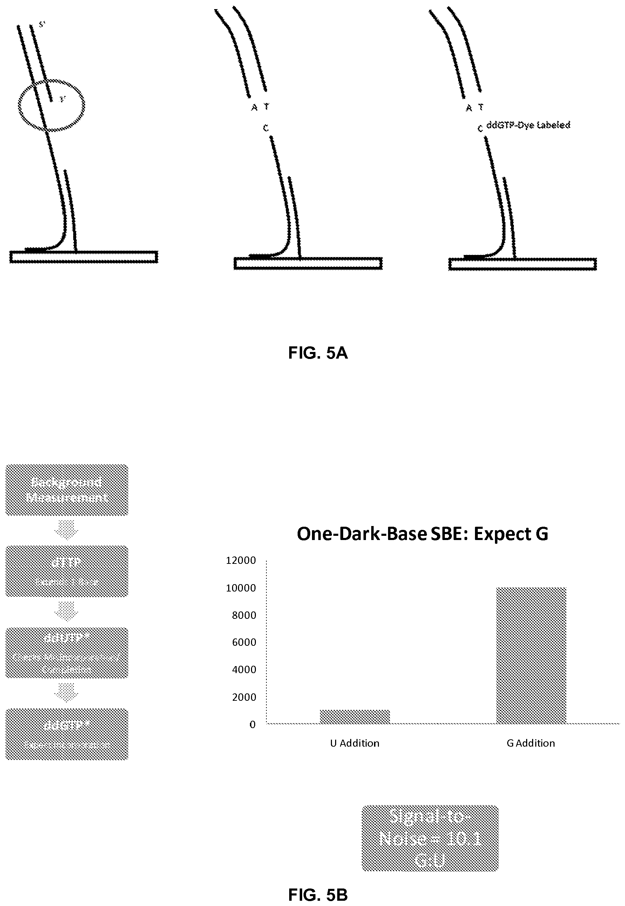

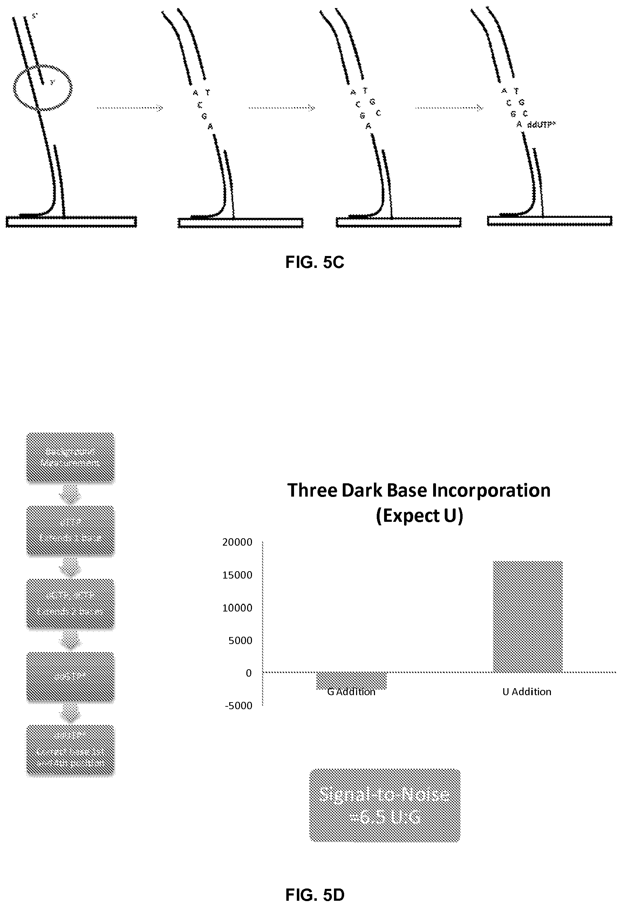

**FIG. 5A** depicts an exemplary embodiment of the invention, one-dark base single-base extension. T is a dark (unlabeled) base, and G is a labeled based. **FIG. 5B** depicts the experimental results of one dark base single-base extension. The signal-to-noise ratio between the labeled (G) and unlabeled (U) is 10.1. **FIG. 5C** depicts an exemplary embodiment of the invention, three dark base single-base extension. T, G, and C are dark (unlabeled) bases, and U is a labeled base. **FIG. 5D** depicts the experimental results of three dark base single-base extension. The signal-to-noise ratio between the labeled (U) and unlabeled (G) is 6.5.

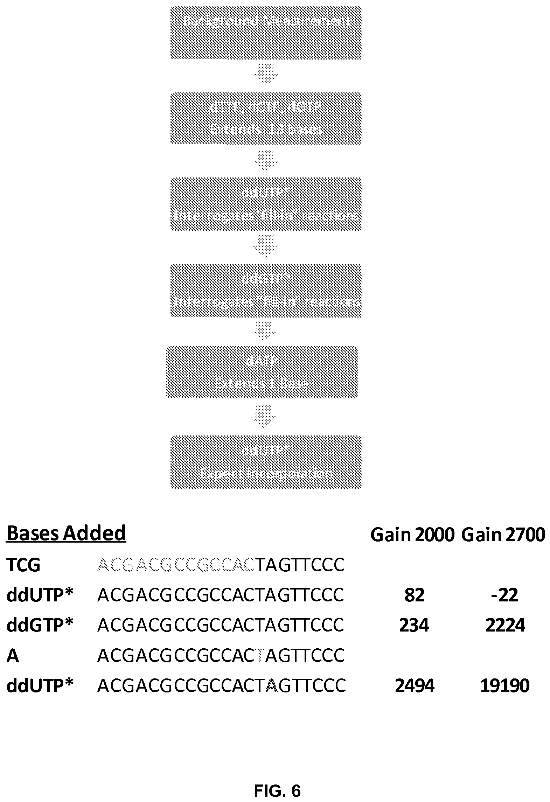

**FIG. 6** depicts an exemplary embodiment of the invention, the incorporation at 15^{th} base (T).

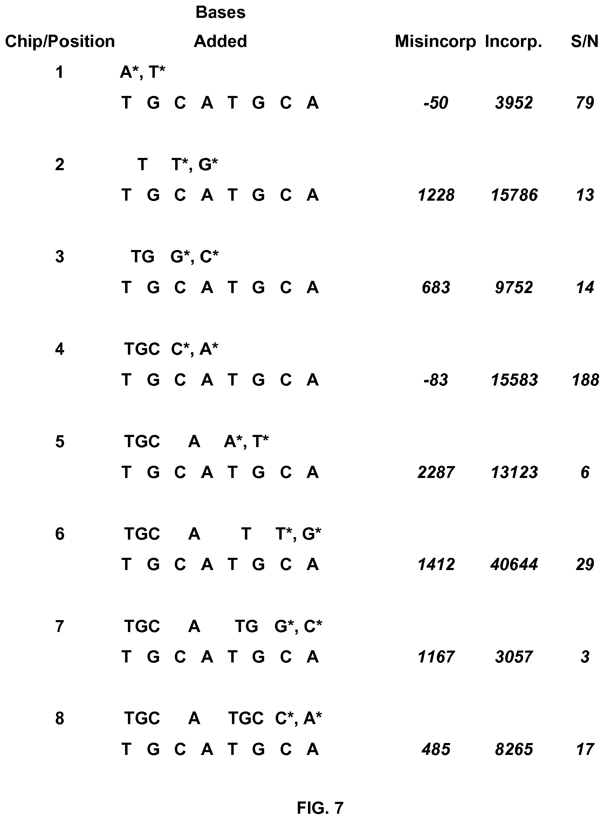

**FIG. 7** depicts an example of eight base read. Incorp.: the expected incorporation; Misincorp.: the mis-incorporation; S/N: signal-to-noise ratio. The same target molecule (8 bases long) is attached to different chips or different positions. For each chip or position, labeled (marked with "*") or dark base (unlabeled) are added as indicated. The signal-noise ratio is between 3 and 188.

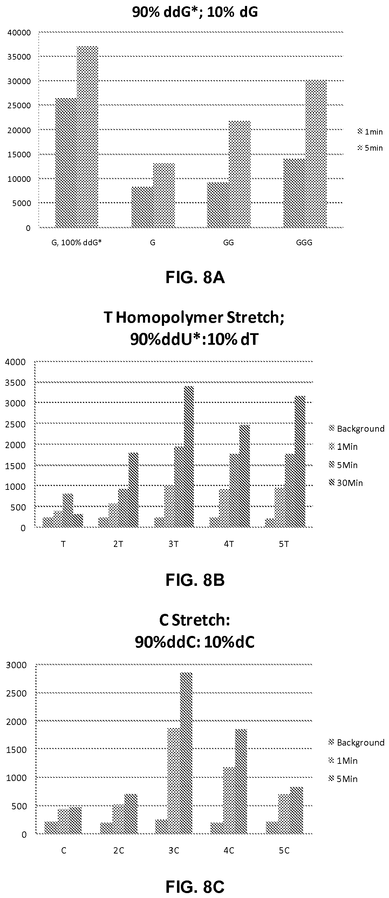

**FIGs. 8A-8C** depict the detection of homopolymers. **8A:** The detection of G stretch. A mixture of labeled ddG and unlabeled ddG (90% v. 10%) was used and the extension time is 1 minute and 5 minutes. **8B:** The detection of T stretch. A mixture of labeled ddU and unlabeled dT (90% v. 10%) was used and the extension time is 1 minute, 5 minutes, and 30 minutes. **8C:** The detection of T stretch. A mixture of labeled ddC and unlabeled dC (90% v. 10%) was used and the extension time is 1 minute, and 5 minutes. The Y axis is the signal intensity and the X axis is the number of bases in a stretch.

### DETAILED DESCRIPTION OF THE INVENTION

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

Such conventional techniques and descriptions can be found in standard laboratory manuals such as Genome Analysis: A Laboratory Manual Series (VoIs. I-IV), Using Antibodies: A Laboratory Manual, Cells: A Laboratory Manual, PCR Primer: A Laboratory Manual, and Molecular Cloning: A Laboratory Manual (all from Cold Spring Harbor Laboratory Press); Stryer, L. (1995) Biochemistry (4th Ed.) Freeman, New York; Gait, "Oligonucleotide Synthesis: A Practical Approach" 1984, IRL Press, London, Nelson and Cox (2000), Lehninger, (2004) Principles of Biochemistry 4th Ed., W. H. Freeman Pub., New York, N.Y. and Berg et al. (2006) Biochemistry, 6th Ed., W. H. Freeman Pub., New York, N.Y., all of which are herein incorporated in their entirety by reference for all purposes.

The present disclosure is directed to compositions and methods for sequencing a target polynucleotide molecule. In general, a set of replicative substrates are generated, each substrates comprises a plurality of capture site and each capture site comprises a capture probe that recognize a target polynucleotide molecule. Preferably, the capture probe on different capture site recognize different target polynucleotide molecule such as different target molecules are sequenced in parallel. However, some of the capture probe on different capture site maybe of the same sequence to provide redundancy.

In the next step, a plurality of target molecules is added to the substrate to form hybridization complexes with the capture probes. As such, each substrate is still a replica of each other. Then different substrates are subjected to different number of base extension to generated staggered fragments, i.e. fragments with increasing length. For example, the first substrate undergoes 0, 1,2, or more cycle of extensions, second substrate undergoes one more extension that the first substrate, the third substrate undergoes one more cycle extension that the second substrate (and thus two more cycle extensions than the first substrate, and so on and so forth. Preferably the first substrate undergoes 0 cycle of extensions. Therefore, if there are n substrates, each is designated as (i), then each substrate under (i-1) number of extension. It is understood each substrate (i) can also undergoes, i, i+1, i+3, etc. cycle of extension. Preferably, the different substrates undergo the extension in parallel.

In the next step (the base extension step), in each extension cycle, one of the dATP, dCTP, dGTP and dTTP is added sequentially, but not necessarily in that order. In some embodiments, the dNTPs are added in different order. In some embodiments, in each extension cycle, two or three NTPs added followed by one or more rrounds of two or more different NTPs, till all four dNTPS are added at least one, thus enable longer extension per extension cycle. After the base extension step, a series of staggered fragments are generated on different substrates that are complement to the same target molecules but are different by one or more bases in length.

In the next step (the base detection step) the series of staggered fragments are further extended (thus, sever as sequencing primer) for sequencing reactions to obtain the sequence information of the target molecules. The sequence information is a series fragment sequences that are adjacent on the target molecule, which can be assembled to obtain a long fragment or the full length sequence of the target molecule.

In one aspect of the invention, serial sequencing of a target polynucleotide is converted to parallel sequencing to reduce the time required for sequencing a given number of bases of the target polynucleotide.

In some embodiments, a primer is hybridized with the target polynucleotide and extension reactions are performed to produce a collection of Extended Primer Sequences complementary to the target polynucleotide. Typically, the collection of Extended Primer Sequences are of different lengths and are produced using the target nucleotide as a template in a controlled fashion. The extension reactions used to produce the collection of Extended Primer Sequences are typically conducted with native nucleotides (as used herein, "native nucleotides" are nucleotides are naturally occurring or modified nucleotides that have similar incorporation efficiency as naturally occurring nucleotides) and a suitable polymerase.

Different extended primer sequences can be separated in a variety of ways such as in different locations of a substrate, in different reaction tubes (e.g., 96 well plate) or in different substrates. At least one of the extended primer sequences can be used to sequence one or more bases (1-20 bases preferred) using the target polynucleotide as the template (sequencing the polynucleotides) and the extended primer sequences as primers. Many sequencing reactions are suitable for detecting one or few bases off a primer hybridized with a template. For example, sequencing with labeled reversible terminators (commercially available from Helicos, Inc., Cambridge, MA, or Illumina,Inc., San Diego, CA), single base extension using labeled dideoxyribonucleotide for DNA sequencing, ligation reaction using labeled random nanomer with an interrogation base, etc. In some embodiments, most or all of the extended primer sequences are used to sequence one or more bases of the target polynucleotide. By assembling the short sequencing data, a large section of the target polynucleotide, such as up to about 10, 20, 30, 40, 100, 500, 1,000 bases, can be sequenced.

In general, the sequencing reactions are carried in parallel for the same target molecule and use naturally occurred nucleotides or modified nucleotides that have incorporation efficiency similar to that of naturally occurred nucleotides and thus the method is called Native-Extension Parallel Sequencing (NPS).

The sequencing method provided by the present invention can be used to sequence DNA/RNA. It can used to sequence pathogens/microbial genomes to identify species/strains quickly. One advantage of the sequencing method provided by the present invention is that is can accommodate low efficiency sequencing chemistry (reversible terminators, ligations, etc.), thus reduces the time to sequence. In addition, the method can sequence very long fragments (e.g. 100-10000 base pairs or more).

The sequencing method provided by the present invention converts serial sequencing to parallel sequencing. In a series of parallel reactions, each reaction extend the DNA (e.g. a capture probe or a primer) at different length to create staggered sequences off a template (the target polynucleotide molecule) using native or native performance nucleotides and polymerase. This is followed by determining a sequence of one or more bases (1-20 preferred) by further extending the staggered sequences in the present of labeled nucleotides. The sequence of the target polynucleotide molecule is obtained by assembling the original template sequence using short sequences from the staggered sequences.

### I. Staggered Base Extension and Base Detection

### Target Preparation

In one aspect, the present invention provides a method for sequencing a target nucleic acid molecule.

By "target nucleic acid molecule", "target molecule", "target polynucleotide", "target polynucleotide molecule" or grammatically equivalent thereof, herein is meant a nucleic acid of interest. In one aspect, target nucleic acids of the invention are genomic nucleic acids. DNA derived from the genetic material in the chromosomes of a particular organism is genomic DNA. A genomic library is a collection of clones made from a set of randomly generated overlapping DNA fragments representing the entire genome of an organism. Target nucleic acids include naturally occurring or genetically altered or synthetically prepared nucleic acids (such as genomic DNA from a mammalian disease model). Target nucleic acids can be obtained from virtually any source and can be prepared using methods known in the art. For example, target nucleic acids can be directly isolated without amplification, isolated by amplification using methods known in the art, including without limitation polymerase chain reaction (PCR), whole genome amplification (WGA), multiple displacement amplification (MDA), rolling circle amplification (RCA), rolling circle amplification (RCR) and other amplification methodologies. Target nucleic acids may also be obtained through cloning, including cloning into vehicles such as plasmids, yeast, and bacterial artificial chromosomes.

In the present invention, a target molecule can be a fragment of genomic DNA that is extracted from an organism (e.g. a cell or bacteria) without any amplification, or a DNA fragment obtained by amplification from a genomic DNA (e.g. a fragment of a genomic DNA library, or a PCR product). The target molecule may also have an exogenous sequence, such as a universal primer sequence or barcode sequence introduced during the amplification process.

In some embodiments, the target polynucleotide is genomic DNA or a part of the genomic DNA. While some embodiments are for sequencing whole genome, such as at more than 50% coverage, these embodiments are also suitable for sequencing a targeted region such as genomic regions relating to drug metabolism In one example, the target polynucleotide is human genomic DNA.

"Nucleic acid" or "oligonucleotide" or "polynucleotide" or grammatical equivalents typically refer to at least two nucleotides covalently linked together. A nucleic acid of the present invention will generally contain phosphodiester bonds, although in some cases, as outlined below (for example in the construction of primers and probes such as label probes), nucleic acid analogs are included that may have alternate backbones, comprising, for example, phosphoramide (Beaucage et al., Tetrahedron 49(10):1925 (1993) and references therein; Letsinger, J. Org. Chem. 35:3800 (1970); Sprinzl et al., Eur. J. Biochem. 81:579 (1977); Letsinger et al., Nucl. Acids Res. 14:3487 (1986); Sawai et al, Chem. Lett. 805 (1984), Letsinger et al., J. Am. Chem. Soc. 110:4470 (1988); and Pauwels et al., Chemica Scripta 26:141 91986)), phosphorothioate (Mag et al., Nucleic Acids Res. 19:1437 (1991); and U.S. Pat. No. 5,644,048), phosphorodithioate (Briu et al., J. Am. Chem. Soc. 111:2321 (1989), O-methylphophoroamidite linkages (see Eckstein, Oligonucleotides and Analogues: A Practical Approach, Oxford University Press), and peptide nucleic acid (also referred to herein as "PNA") backbones and linkages (see Egholm, J. Am. Chem. Soc. 114:1895 (1992); Meier et al., Chem. Int. Ed. Engl. 31:1008 (1992); Nielsen, Nature, 365:566 (1993); Carlsson et al., Nature 380:207 (1996). Other analog nucleic acids include those with bicyclic structures including locked nucleic acids (also referred to herein as "LNA"), Koshkin et al., J. Am. Chem. Soc. 120.13252 3 (1998); positive backbones (Denpcy et al., Proc. Natl. Acad. Sci. USA 92:6097 (1995); non- ionic backbones (U.S. Pat. Nos. 5,386,023, 5,637,684, 5,602,240, 5,216,141 and 4,469,863; Kiedrowshi et al., Angew. Chem. Intl. Ed. English 30:423 (1991); Letsinger et al., J. Am. Chem. Soc. 110:4470 (1988); Letsinger et al., Nucleoside & Nucleotide 13:1597 (1994); Chapters 2 and 3, ASC Symposium Series 580, "Carbohydrate Modifications in Antisense Research", Ed. Y. S. Sanghui and P. Dan Cook; Mesmaeker et al., Bioorganic & Medicinal Chem. Lett. 4:395 (1994); Jeffs et al., J. Biomolecular NMR 34:17 (1994); Tetrahedron Lett. 37:743 (1996)) and non-ribose backbones, including those described in U.S. Pat. Nos. 5.235,033 and 5,034,506, and Chapters 6 and 7, ASC Symposium Series 580, "Carbohydrate Modifications in Antisense Research", Ed. Y. S. Sanghui and P. Dan Cook. Nucleic acids containing one or more carbocyclic sugars are also included within the definition of nucleic acids (see Jenkins et al., Chem. Soc. Rev. (1995) pp 169 176). Several nucleic acid analogs are described in Rawls, C & E News Jun. 2, 1997 page 35. "Locked nucleic acids" are also included within the definition of nucleic acid analogs. LNAs are a class of nucleic acid analogues in which the ribose ring is "locked" by a methylene bridge connecting the 2'-0 atom with the 4'-C atom. These modifications of the ribose-phosphate backbone may be done to increase the stability and half-life of such molecules in physiological environments. For example, PNA:DNA and LNA-DNA hybrids can exhibit higher stability and thus may be used in some embodiments. The target nucleic acids may be single stranded or double stranded, as specified, or contain portions of both double stranded or single stranded sequence. Depending on the application, the nucleic acids may be DNA (including genomic and cDNA), RNA (including mRNA and rRNA) or a hybrid, where the nucleic acid contains any combination of deoxyribo- and ribo-nucleotides, and any combination of bases, including uracil, adenine, thymine, cytosine, guanine, inosine, xathanine hypoxathanine, isocytosine, isoguanine, etc.

In some embodiments, the methods of the present invention comprise capture of target polynucleotide. The target polynucleotide may be from a known region of the genome. In one embodiment, oligo probes can be immobilized on beads and these oligo beads which are inexpensive and reusable can be used to capture the target genomic polynucleotide. In another embodiment, microarrays are used to capture target polynucleotide.

In some embodiments, target polynucleotide is amplified using standard amplification methods known in the relevant art. In one embodiment, the target polynucleotide is prepared by whole genome amplification (WGA). WGA methods include the ligation-mediated PCR (LMP), the T7-based linear amplification of DNA (TLAD) and the multiple displacement amplification (MDA). LMP is a method that uses endonuclease or chemical cleavage to fragment the gDNA sample and uses linkers and primers for its amplification. It was adapted for the WGA of small quantities of gDNA and single cells (Klein *et al.,* 1999; Tanabe *et al.,* 2003). Rubicon Genomics commercializes different kits (Omniplex) that allow for the amplification of RNA, DNA and methylated DNA sequences. The main advantages are that the method is able to amplify degraded DNA, and allows for different variations and that all steps are performed in the same tube. TLAD is a variant on the protocol originally designed by Phillips and Eberwine to amplify mRNA (Phillips and Eberwine, 1996) that has been adapted for WGA (Liu *et al.,* 2003). It uses Alu I restriction endonuclease digestion and a terminal transferase to add a polyT tail on the 3' terminus. A primer is then used with a 5' T7 promoter and a 3' polyA tract, and Taq polymerase is used to synthesize the second strand. Then the sample is submitted to *in vitro* transcription reaction and posterior reverse transcription. The major advantage is that TLAD does not introduce sequence and length-dependent biases. Multiple displacement amplification (MDA) is a non-PCR-based isothermal method based on the annealing of random hexamers to denatured DNA, followed by strand-displacement synthesis at constant temperature (Blanco *et al.,* 1989). It has been applied to small genomic DNA samples, leading to the synthesis of high molecular weight DNA with limited sequence representation bias (Lizardi *et al.,* 1998; Dean *et al.,* 2002). As DNA is synthesized by strand displacement, a gradually increasing number of priming events occur, forming a network of hyper-branched DNA structures. The reaction can be catalyzed by the Phi29 DNA polymerase or by the large fragment of the Bst DNA polymerase. The Phi29 DNA polymerase possesses a proofreading activity resulting in error rates 100 times lower than the Taq polymerase (Eckert and Kunkel, 1991; Esteban *et al.,* 1993). Recently, it has been shown that MDA, when used on genomic DNA sequences with high variability, results in a loss of heterozygosity (Murthy *et al.,* 2005). The technology has been shown to be very sensitive and can amplify from single cells (Hellani et al., 2004, Handyside et al., 2005) and single bacteria (Raghunathan et al., 2005). Any of the herein disclosed method can be used in the methods of the present invention.

In another embodiment, the target polynucleotide is prepared by whole genome sampling assay (WGSA). The WGSA reduces the complexity of a nucleic acid sample by amplifying a subset of the fragments in the sample. A nucleic acid sample is fragmented with one or more restriction enzymes and an adapter is ligated to both ends of the fragments. A primer that is complementary to the adapter sequence is used to amplify the fragments using PCR. During PCR fragments of a selected size range are selectively amplified. The size range may be, for example, 400-800 or 400 to 2000 base pairs. Fragments that are outside the selected size range are not efficiently amplified. The fragments that are amplified by WGSA may be predicted by in silico digestion and an array may be designed to genotype SNPs that are predicted to be amplified. Genotyping may be done by allele specific hybridization with probes that are perfectly complementary to individual alleles of a SNP. A set of probes that are complementary to the region surrounding each SNP may be present on the array. Perfect match probes are complementary to the target over the entire length of the probe. Mismatch probes are identical to PM probes except for a single mismatch base. The mismatch position is typically the central position. WGSA is disclosed in Kennedy et al. (2003), Nat Biotechnol , Vol., pp.1233-1237, and U.S. patent application Ser. Nos. 09/920,492, 09/904,039, 10/681,773, 10/316,517, 10/442,021, 10/463,991, 10/316, 629, and 10/264,945 and U.S. Pat. No. 6,361,947. WGSA can simultaneously genotype more than 10,000 SNPs in parallel by allele-specific hybridization to perfect match (PM) and mismatch (MM) probes synthesized on an array. WGSA may not be able to assay the entire panels of loci.

In another embodiment, the target polynucleotide is prepared by long-range PCR. Long range PCR allows the amplification of PCR products, which are much larger than those achieved with conventional *Taq* polymerases. Up to 27 kb fragments are possible from good quality genomic DNA, although 10 - 20 kb fragments are routinely achievable, given the appropriate conditions. The method relies on a mixture of thermostable DNA polymerases, usually *Taq* DNA polymerase for high processivity (i.e. 5'-3' polymerase activity) and another DNA polymerase with 3'-5' proofreading abilities (usually Pwo). This combination of features allows longer primer extension than can be achieved with *Taq* alone. This method for detection of the FVIII gene intron 22 inversion (Liu *et al,* 1998) removes the requirement for Southern Blotting. Results can be obtained within 24 hours. Modifications from standard long range PCR protocols include the addition of DMSO and incorporation of deaza GTP to enable read through of a high GC content region upstream of the FVIII gene. The method relies on overlapping PCR to generate a constant band, which appears in all template DNA's. This band acts as a control to show that the reaction has worked efficiently. The largest amplification product seen using this method is 12 kb, well within the range of the enzyme mix utilized. Long-range PCR may be costly to assay singleton loci that are distant from groups of clustered loci.

In another embodiment, the target polynucleotide is prepared by locus-specific multiplex PCR. Multiplex locus specific amplification can be used to amplify a plurality of pre-selected target sequences from a complex background of nucleic acids. The targets are selected for amplification using splint oligonucleotides that are used to modify the ends of the fragments. The fragments have known end sequences and the splints are designed to be complementary to the ends. The splint can bring the ends of the fragment together and the ends are joined to form a circle. The splint can also be used to add a common priming site to the ends of the target fragments. Specific loci are amplified and can be subsequently analyzed.

Other suitable amplification methods include but are not limited to the ligase chain reaction (LCR) (e.g., Wu and Wallace, Genomics 4, 560 (1989), Landegren et al., Science 241, 1077 (1988) and Barringer et al. Gene 89:117 (1990)), transcription amplification (Kwoh et al., Proc. Natl. Acad. Sci. USA 86, 1173 (1989) and WO88/10315), self-sustained sequence replication (Guatelli et al., Proc. Nat. Acad. Sci. USA, 87, 1874 (1990) and WO90/06995), selective amplification of target polynucleotide sequences (U.S. Pat. No. 6,410,276), consensus sequence primed polymerase chain reaction (CP-PCR) (U.S. Pat. No. 4,437,975), arbitrarily primed polymerase chain reaction (AP-PCR) (U.S. Pat. Nos. 5,413,909, 5,861,245) and nucleic acid based sequence amplification (NABSA). (See, U.S. Pat. Nos. 5,409,818, 5,554,517, and 6,063,603). Other amplification methods that may be used are described in, U.S. Pat. Nos. 5,242,794, 5,494,810, 4,988,617 and in U.S. Ser. No. 09/854, 317. Additional methods of sample preparation and techniques for reducing the complexity of a nucleic sample are described in Dong et al., Genome Research 11, 1418 (2001), in U.S. Pat. Nos. 6,361,947, 6,391,592 and U.S. Ser. Nos. 09/916,135, 09/920,491, 09/910,292, and 10/013,598.

Naturally-existing targets can be assayed directly in cell lysates, in nucleic acid extracts, or after partial purification of fractions of nucleic acids so that they are enriched in targets of interest. In one example, the target polynucleotide is human genomic DNA. The polynucleotide target to be detected can be unmodified or modified. Useful modifications include, without limitation, radioactive and fluorescent labels as well as anchor ligands such as biotin or digoxigenin. The modification(s) can be placed internally or at either the 5' or 3' end of the targets. Target modification can be carried out post-synthetically, ether by chemical or enzymatic reaction such as ligation or polymerase- assisted extension. Alternatively, the internal labels and anchor ligands can be incorporated into an amplified target or its complement directly during enzymatic polymerization reactions using small amounts of modified NTPs as substrates.

The target polynucleotide can be isolated from a subject. The subject is not limited to a human being but may also be other organisms including but not limited to mammals, plants, bacteria, virus or fungi. In one example, the target polynucleotide is genomic DNA extracted from a human.

In some embodiments, the target polynucleotide may be fragmented to suitable length such as approximately between 100-200, 200-300, 300-500, 500-1000, 1000-2000 bases in length.

In yet another embodiment, target polynucleotides are produced using multiplex PCR and each of the PCR fragments is labeled with a tag sequence. Such tag sequence can be added as a part of one of the primers used for the PCR. Therefore, each resulting PCR fragment can be uniquely identified. Such applications are particularly useful for the identification of microbial species.

### Methods of Immobilizing Target Polynucleotides

Some embodiments of the invention employs replicate substrates where target polynucleotides are immobilized. For sequencing multiple target polynucleotides (or fragments of polynucleotide targets), a large number of different target polynucleotides or its fragments are immobilized on a substrate. Such a substrate is replicated many times to produce a collection of the substrates.

In some embodiments of the invention, the replicate substrates are microarrays with capture probes. Target samples are hybridized with a set of functionally identical microarrays to produce a set of substrates with each of the target polynucleotide molecules are in identifiable or specific locations.

In some embodiments, the methods of the invention comprise capture probes, generally stretched on a substrate. By "capture probe" herein is meant an oligonucleotide that is attached to the surface of a substrate and is capable to bind to a target molecule. Capture probe of the invention can be of various lengths, from 18 bases to 100 bases, preferably 20 bases to 50 bases.

In some embodiments, the capture probe has a sequence that is complement to the target molecule. For example, if the present method is used to sequence a genome with at least partial sequence known already, capture probes can be designed to complement to the known sequences. In some other embodiments, the capture probes are complementary to "barcode" or "identifier" sequence added to target polynucleotide via, e.g., specific ligation, as a part of the primer for PCR reaction, etc. In such reaction, a target molecule specific primer and a primer comprises a unique barcode are used for the amplification, thus all the target molecules with the same sequences have the same barcode attached.

The capture probe can be attached to the substrate at either 5' end or 3' end. Preferably, the capture probe is attached to the substrate at the 5'end and the '3 end of the capture probe can extended by the incorporation of nucleotides as described herein to generate staggered extension fragment which can in turn be sequenced by further incorporation of labeled nucleotide.

In some embodiments, the capture probe is attached to the substrate at the 3'end and the '3 end of the capture probe cannot be extended by the incorporation of nucleotides. A second probe (also known as sequencing primer) hybridizes to the target molecule and its 3'end is extended by the incorporation of nucleotides as described herein to generate staggered extension fragment which can in turn be sequenced by further incorporation of labeled nucleotide. In this case, the extension is towards the direction of the capture probe. In general, the sequencing primer hybridizes to a linker introduced to the end of the target molecule when the target molecule as generated, either direct from a genome DNA or a parent target molecule. Thus the sequencing is a "universal primer" that can used to sequence different target molecules. In some embodiments, sequencing primer that specific to the target molecule can be used, but this is not preferred in some embodiments due to increasing cost of primer synthesis.

The terms "substrate" or "solid support" or other grammatical equivalents as used herein typically refer to any material that is modified to allow "stretching" of nucleic acid molecules as described herein. In general, the substrate contains discrete individual sites (for example, nanochannels, flow cells, or lines) appropriate for the attachment or association of decorated nucleic acid molecules to form stretched nucleic acids and is amenable to at least one detection method. As will be appreciated by those in the art, the number of possible substrates is very large. Possible substrates include, but are not limited to, glass and modified or functionalized glass, plastics (including acrylics, polystyrene and copolymers of styrene and other materials, polypropylene, polyethylene, polybutylene, polyurethanes, TeflonJ, etc.), polysaccharides, nylon or nitrocellulose, resins, silica or silica-based materials including silicon and modified silicon, carbon, metals, inorganic glasses, plastics, optical fiber bundles, and a variety of other polymers. In general, the substrates allow optical detection and do not appreciably fluoresce themselves.

Substrates of the invention can be configured to have any convenient geometry or combination of structural features. The substrates can be either rigid or flexible and can be either optically transparent or optically opaque, or have combinations of these surfaces. The substrates can also be electrical insulators, conductors or semiconductors. Further the substrates can be substantially impermeable to liquids, vapors and/or gases or, alternatively, the substrates can be substantially permeable to one or more of these classes of materials. In general, the substrates fall into two different classes: substrates comprising particular geometries such as nanochannels or nanopores, as more fully discussed below, or those that have surface characteristics to allow the stretching of decorated nucleic acids, such as the use of linear patterns of surface chemistries.

In one aspect of the invention, substrates of the invention comprise nanostructures or cells. Such structures can include without limitation nanopillars, nanopores and nanochannels. In many exemplary aspects, substrates of the invention comprise nanochannels. Such substrates are known in the art. For example, U.S. Patent Nos. 7,217,562; 6,685,841; 6,518,189; 6,440,662; 6,214,246 describe nanostructures, including nanochannels, of use in accordance with the present invention. Generally, in these nanochannel substrates, there is a reservoir into which the oligonucleotide probes are placed, which are then moved into nanochannels, a single molecule of oligonucleotide probe per nanochannel, to form the stretched nucleic acids, followed by detection of the order, and optionally, the distance between the labels of the incorporated probes.

In some embodiments, the substrates comprise cells that are generally 1-2 millimeters thick. In one example, the substrate, e.g. a slide can be about 10 centimeters long. Another embodiment of nanostructures that finds use in the present invention are substrates comprising nanopores. Nanopore devices can provide single-molecule detection of molecules driven electrophoretically in solution through a nano-scale pore, and the sequence of nucleotides can be detected by the sequence of signals generated as each nucleotide passes through the pore. Such nanopores and methods of sequencing using nanopores are known in the art and discussed in for example, Branton et al., (2008), Nature, 26(10):1 146-53 and in U.S. Patent Nos. 6,673,615; 7,258,838; 7,238,485; 7,189,503;6,627,067; 6,464,842; 6,267,872 and U.S. Patent Application Nos. 20080248561; 20080171316, 20080102504.

In some embodiments, the oligonucleotide probe is immobilized on a solid support before binding to the target polynucleotide. In one embodiment, the 5' end of an oligonucleotide probe of the present invention is attached to a solid surface or substrate. Oligonucleotide can be immobilized by various methods known in the art including, (without limitation) covalent cross-linking to a surface (e.g., photochemically or chemically), non-covalent attachment to the surface through the interaction of an anchor ligand with a corresponding receptor protein (e.g. biotin- streptavidin or digoxigenin-anti-digoxigenin antibody), or through hybridization to an anchor nucleic acid or nucleic acid analog. The anchor nucleic acid or nucleic acid analog have sufficient complementarity to the target (i.e., their formed duplex has sufficiently high Tm) that the anchor-target-probe complex will survive stringent washing to remove unbound targets and probes, but they do not overlap with the target site that is complementary to the probe antisense sequence.

The solid substrate can be made of any material to which the molecules can be bound, either directly or indirectly. Examples of suitable solid substrates include flat glass, quartz, silicon wafers, mica, ceramics and organic polymers such as plastics, including polystyrene and polymethacrylate. The surface can be configured to act as an electrode or a thermally conductive substrate (which enhances the hybridization or discrimination process). For example, micro and sub-micro electrodes can be formed on the surface of a suitable substrate using lithographic techniques. Smaller nanoelectrodes can be made by electron beam writing/lithography. Electrodes can also be made using conducting polymers which can be pattern a substrate by ink-jet printing devices by soft lithography or be applied homogenously by wet chemistry. TnO₂ coated glass substrates are available. Electrodes can be provided at a density such that each immobilized molecule has its own electrode or at a higher density such that groups of molecules or elements are connected to an individual electrode. Alternatively, one electrode may be provided as a layer below the surface of the array which forms a single electrode. The solid substrate may optionally be interfaced with a permeation layer or a buffer layer. It is also possible to use semi-permeable membranes such as nitrocellulose or nylon membranes, which are widely available. The semi-permeable membranes can be mounted on a more robust solid surface such as glass. The surface layer may comprise a sol-gel. The surfaces may optionally be coated with a layer of metal, such as gold, platinum or other transition metal. A particular example of a suitable solid substrate is the commercially available SPR BIACore™ chip (Pharmacia Biosensors). Heaton et al., 2001 (PNAS 98:3701-3704) have applied an electrostatic field to an SPR surface and used the electric field to control hybridization.

The solid substrate is generally a material having a rigid or semi-rigid surface. In some embodiments, at least one surface of the substrate is substantially flat, although in some embodiments it may be desirable to physically separate discrete elements with, for example, raised regions or etched trenches. For example, the solid substrate may comprise nanovials-small cavities in a flat surface e.g. 10 µm in diameter and 10 µm deep. This is particularly useful for cleaving molecules from a surface and performing assays or other processes such as amplification in them. The solution phase reaction is more efficient than the solid phase reaction, whilst the results remains spatially addressable, which is advantageous. Other formats include but are not limited to synthetic or natural beads, membranes or filters, slides including microarray slides, microtiter plates, microcapillaries, and microcentrifuge tubes.

In some embodiments, the loci-specific oligo probes are coated or attached onto beads for capturing genomic DNA. The oligo probes can be directed against large regions on genomic DNA that include multiple loci of interest. For example, many ADME (absorption, distribution, metabolism, and excretion) markers are on about 200 genes. Hybridization between loci-specific oligo probes and target polynucleotide can be carried out on beads in columns at a controlled temperature and salt concentration. The hybridization products can be eluted from the beads with moderate pressure.

The use of a solid support with an array of capture oligonucleotides is disclosed in U.S. Patent Application Ser. No. 60/011,359. When using such arrays, the oligonucleotide primers or probes used in the above-described coupled PCR and LDR phases, respectively, have an addressable array-specific portion. After the LDR or PCR phases are completed, the addressable array-specific portions for the products of such processes remain single stranded and are caused to hybridize to the capture oligonucleotides during a capture phase. C. Newton, et al., "The Production of PCR Products With 5' Single-Stranded Tails Using Primers That Incorporate Novel Phosphoramidite Intermediates," Nucl. Acids Res. 21(5): 1155-62 (1993).

During the capture phase of the process, the mixture can be contacted with the solid support at a temperature of 45-90°C and for a time period of up to 60 minutes. Hybridizations may be accelerated by adding cations, volume exclusion or chaotropic agents. When an array consists of dozens to hundreds of addresses, it is important that the correct ligation product sequences have an opportunity to hybridize to the appropriate address. This may be achieved by the thermal motion of oligonucleotides at the high temperatures used, by mechanical movement of the fluid in contact with the array surface, or by moving the oligonucleotides across the array by electric fields. After hybridization, the array is washed sequentially with a low stringency wash buffer and then a high stringency wash buffer.

Loading of nucleic acids onto these substrates can be modulated and/or controlled by the flow and/or electrical forces, including diffusion forces and surface forces exerted by areas of differential charge and/or hydrophobicity. The number of nucleic acids applied to the substrate (i.e., with a loading buffer or other solution) can be adjusted to assure maximal occupancy of the linear features with nonoverlapping nucleic acid molecules and thus minimize the number of empty linear features on the substrate. In an exemplary embodiment, at least 50% of the linear features of a substrate are occupied by at least one nucleic acid molecule. In a further embodiment, at least 60%, 70%, 80%, 90%, and 95% of the linear features are occupied by one or more nucleic acids.

Two exemplary approaches of laying probes are disclosed herein below for illustrative purposes. The first approach is "In Situ" oligonucleotide synthesis in which the probes are in known geographic locations in the X-Y coordinate plane. In one embodiment, the oligonucleotide probe is synthesized on the surface. Examples of technologies that allow on-surface oligo synthesis include but are not limited to photolithography and ink jet. In another embodiment, the pre-synthesized oligonucleotide probes are spotted onto the surface. Various microarray protocols, for example, protocol for Agilent inkjet-deposited pre-synthesized oligo arrays are known to one skilled in the art.

Polymers such as nucleic acids or polypeptides can be synthesized in situ using photolithography and other masking techniques whereby molecules are synthesized in a step-wise manner with incorporation of monomers at particular positions being controlled by means of masking techniques and photolabile reactants. For example, U.S. Pat. No. 5,837,832 describes a method for producing DNA arrays immobilized to silicon substrates based on very large scale integration technology. In particular, U.S. Pat. No. 5,837,832 describes a strategy called "tiling" to synthesize specific sets of probes at spatially-defined locations on a substrate. U.S. Pat. No. 5,837,832 also provides references for earlier techniques that can also be used. Light directed synthesis can also be carried out by using a Digital Light Micromirror chip (Texas Instruments) as described (Singh-Gasson et al., (1999) Nature Biotechnology 17:974-978). Instead of using photo-deprotecting groups which are directly processed by light, conventional deptotecting groups such as dimethoxy trityl can be employed with light directed methods where for example a photoacid is generated in a spatially addressable way which selectively deprotects the DNA monomers (McGall et al PNAS 1996 93: 1355-13560; Gao et al J. Am. Chem Soc. 1998 120: 12698-12699). Electrochemical generation of acid is another means that can be used in the subject methods of the present invention.

The "in situ" arrays can have about 1,000 to 100,000,000 array probes (features). In one embodiment, the "in situ" array carries approximately 200,000,000 probes.

Molecules that can be immobilized in the array include nucleic acids such as DNA and analogues and derivatives thereof, such as PNA. Nucleic acids can be obtained from any source, for example genomic DNA or cDNA or synthesized using known techniques such as step-wise synthesis. Nucleic acids can be single or double stranded. DNA nanostructures or other supramolecular structures can also be immobilized. Other molecules include but are not limited to compounds joined by amide linkages such as peptides, oligopeptides, polypeptides, proteins or complexes containing the same; defined chemical entities, such as organic molecules; conjugated polymers and carbohydrates or combinatorial libraries thereof.

Molecules can be labeled to enable interrogation using various methods. Suitable labels include: optically active dyes, such as fluorescent dyes; nanoparticles such as fluorospheres and quantum dots, rods or nanobars; and surface plasmon resonant particles (PRPs) or resonance light scattering particles (RLSs)-particles of silver or gold that scatter light (the size and shape of PRP/RLS particles determines the wavelength of scattered light). See Schultz et al., 2000, PNAS 97: 996-1001; Yguerabide, J. and Yguerabide E., 1998, Anal Biochem 262: 137-156.

### Hybridization

In one aspect, the present invention provides a method for sequencing a target polynucleotide comprising the step of: forming a plurality of hybridization complexes on a substrate, each complex comprises: a capture probe and a copy of the target nucleic acid molecule.

In general, there are a plurality of substrates and the total number of substrates (e.g. chips) is n which is an integer from 10 to 100, or more. Each substrate is designated as substrate (i), wherein i is an integer from 1 to n, inclusive. For example, if there are five chips, they are designated as chip 1, 2, 3, 4, and 5. See FIG. 1A. It should be understood that the assignment of a number to each substrate is for convenience purpose only. Alternative system can be used that is consistent with the present invention.

In some embodiments, a target molecule is amplified to generate copies of it, such as by clonal amplification, and one or more copies of the target molecules are hybridized to capture probes that are also replicates but located on different substrates, thus allow parallel extension and detection as provided herein. Thus a sample comprises the target molecules or copies thereof are used to be in contact with the substrates. In some embodiments, each substrate is located in separate reaction chambers and the sample is divided to be distributed among the different reaction chambers. In some embodiments, the substrates are located in the same reaction chambers and the sample is load and the target molecules are captured by the capture probes on different substrates. Alternatively, no amplification is used. Single molecule can often generate sufficient signal for sequencing reaction as described below.

In some embodiments, a plurality of hybirdization complexs are formed on different substrates, each complexe has capture probe of identical sequences and target molecules of identical sequences. Thus, the different hybridization complexes formed on differnt substrates are "replicate" of each other. However, it is understood that each capure probe and targat molecule on different susbtates may have sequence variation, as long as the complementary sequences between the caputure probe and the target molecules are the same to allow parallel extension, detection and the eventual assembly of the sequence reads from different substrate to obtain the sequence information of the target molecule.

In some embodiments, the sequence is conduced by multiplexing with each substate has a plurality of capture sites and each capture sites has different capture probe. However, there are corresponding capture sites on each substrate that have identical (or substantail identical) capture probes attached. In the manner, the substrates are used to generate sequence reads from multiple different target molecules in parallel.

"Hybridization" as used herein typically refers to the technique of allowing two single-stranded polynucleotide sequences with some degree of complementarity to bind to one another to form a stable double-stranded polynucleotide. "Complementary" and its equivalents as used herein generally refers to the hybridization or base pairing between nucleotides or nucleic acids, such as, for instance, between the two strands of a double stranded DNA molecule or between an oligonucleotide primer and a primer binding site on a single stranded nucleic acid to be sequenced or amplified. Complementary nucleotides are, generally, A and T (or A and U), or C and G. Two single stranded RNA or DNA molecules are said to be complementary when the nucleotides of one strand, optimally aligned and compared and with appropriate nucleotide insertions or deletions, pair with at least about 70% of the nucleotides of the other strand, usually at least about 80%, 85%, 90% to 95%, and more preferably from about 98 to 100%. See, M. Kanehisa Nucleic Acids Res. 12:203 (1984).

Hybridization usually involves the steps of 1) allowing binding between probe and target; and 2) washing away unbound or weakly bound probes under stringent conditions, wherein stringent hybridization conditions are those washing conditions that provide dissociation for imperfect complexes while preserving the intended complexes between target-specific probes and corresponding targets. Improvements in hybridization characteristics can be improvements in the selectivity of hybridization (sequence specificity and mismatch discrimination), the sensitivity of hybridization (ratio of absolute signal to background signal, signal-to-noise ratio), the affinity between probe and target (ratio of binding rate to dissociation rate between hybridization probes and targets); the stability of the duplex or complex (thermal stability, Tm; also kinetic inertness of dissociation or kinetic trap), or the efficiency or efficacy of hybridization (hybridization rate and/or yield of complex between probe and target for a fixed time of incubation under hybridization conditions). Methods for conducting polynucleotide hybridization assays have been well developed in the art. Hybridization assay procedures and conditions will vary depending on the application and are selected in accordance with the general binding methods known including those referred to in: Maniatis et al. Molecular Cloning: A Laboratory Manual (2nd Ed. Cold Spring Harbor, N.Y, 1989); Berger and Kimmel Methods in Enzymology, Vol. 152, Guide to Molecular Cloning Techniques (Academic Press, Inc., San Diego, Calif., 1987); Young and Davism, P.N.A.S, 80: 1194 (1983). Methods and apparatus for carrying out repeated and controlled hybridization reactions have been described in U.S. Pat. Nos. 5,871,928, 5,874,219, 6,045,996 and 6,386,749, 6,391, 623.

In some embodiments, the primary polymerase chain reaction mixture is subjected to two or more polymerase chain reaction cycles involving a denaturation treatment, a hybridization treatment, and an extension treatment. During hybridization, target-specific portions of the probe hybridize to the target nucleotide sequences. The extension treatment causes hybridized primary oligonucleotide primers to be extended to form primary extension products complementary to the target nucleotide sequence to which the primary oligonucleotide primers are hybridized.

In some embodiments, the hybridization probe is allele-specific and the sequence of the probe is known so that the capture oligonucleotide probe can hybridize with the target polynucleotide sequence in a stable fashion. Unless the oligonucleotides are designed in this fashion, false positive signals may result due to capture of adjacent unreacted oligonucleotides from the same oligonucleotide set which are hybridized to the target. In one aspect, the ligation step with the solution probes and the subsequent capping of the 3' end of the hybridization products and the cleaving of the 3' end cap of the specific probe-target hybridization complexes allow for higher specificity of hybridization to be achieved. In some embodiments, the hybridization specificity is greater than 95%, 96%, 97%, 98%, 99%, 99.5% or higher.

The stringency for target-probe hybridization can be adjusted and optimized. Hybridization stringency typically refers to the degree to which mismatches are tolerated in a hybridization assay. High stringency is achieved by using a high temperature and low salt concentration. Increasing the concentration of salt and reducing the temperature reduces the hybridization stringency, and enhances the stability of mismatched heteroduplexes. In some embodiments of the present invention, the highest possible stringency in hybridization and washing is used in the subject methods to increase hybridization specificity. In some embodiments, nonspecific hybridization products between a capture probe and the target polynucleotide can be washed off with high-stringency washing. The ionic strength of the buffers used for washing can be adjusted, for example, salt concentration can be lowered for extreme stringency. In some embodiments, the target polynucleotide, for example, genomic DNA can be washed off of the hybridization duplex to allow subsequent primer annealing and base extension.

### Native Base Extension

In some embodiments, target polynucleotides on each of the replicate substrates are hybridized with a sequence primer. At least one the replicate substrates is contacted with native nucleotide mixture and a polymerase for extension. The nucleotide mixture can contain one, two or three different native nucleotides (such as a mixture dATP, dCTP, dGTP) or their equivalents. Therefore, the extension reaction is controlled and can not extend beyond where the missing base(s) is needed. For example, if the nucleotide mixture contains dATP, dCTP, and dGTP, the extension reaction will stop at the position where the target polynucleotide calls for a dTTP. There are a number of different ways to control the extension reaction. For example, sequential addition of dATP, dCTP, dGTP, and dTTP (one base cycle with four steps of nucleotide addition; specific sequence of base is not important) with washing steps in between (or nucleotide degradation steps in between) will extend at least one base per cycle. On average, such a cycle generates about 2 base extension per cycle for a typical genome. Sequential addition of three bases (e.g., dATP/dCTP/dGTP, dCTP/dGTP/dTTP, dGTP/dTTP/dATP, dATP/dCTP/dTTP with four cycles of nucleotide addition) is a way to extend more bases per reaction, but still with control and synchronization.

In some embodiments, each of the replicate substrate undergoes different steps of extension. Therefore, one cycle (four steps) may produce four different replicate substrates. After extension, the replicate plates may or may not be different in terms of primer length. However, after one cycle of one base addition, at least one of the replicate substrate will have different primer length. Similarly, at least one is extended after two steps of extension in a three base cycle and at least one replicate plate has different primer length from others.

In general, the primers used according to the methods of the invention embrace oligonucleotides of sufficient length and appropriate sequence which provide specific initiation of polymerization of a significant number of nucleic acid molecules containing the target nucleic acid under the conditions of stringency for the reaction utilizing the primers. I n this manner, it is possible to selectively amplify the specific target nucleic acid sequence containing the nucleic acid of interest. Specifically, the term "primer" as used herein refers to a sequence comprising two or more deoxyribonucleotides or ribonucleotides, preferably at least eight, which sequence is capable of initiating synthesis of a primer extension product that is substantially complementary to a target nucleic acid strand. The oligonucleotide primer typically contains 15-22 or more nucleotides, although it may contain fewer nucleotides as long as the primer is of sufficient specificity to allow essentially only the amplification of the specifically desired target nucleotide sequence (i.e., the primer is substantially complementary). The exact length of primer will depend on many factors, including temperature, buffer, and nucleotide composition. "Substantially complementary" refers to that the primers are sufficiently complementary to hybridize with their respective strands under conditions which allow the agent for polymerization to function. In other words, the primers should have sufficient complementarily with the flanking sequences to hybridize with and permit amplification of the nucleotide sequence. Preferably, the 3' terminus of the primer that is extended has perfectly base paired complementarity with the complementary flanking strand. The oligonucleotide primers for use in the invention may be prepared using any suitable method, such as conventional phosphotriester and phosphodiester methods or automated embodiments thereof. In one such automated embodiment, diethylphosphoramidites are used as starting materials and may be synthesized as described by Beaucage, et al. (Tetrahedron Letters, 22:1859-1862, 1981). One method for synthesizing oligonucleotides on a modified solid support is described in U.S. Pat. No. 4,458,066. One method of amplification which can be used according to this invention is the polymerase chain reaction (PCR) described in U.S. Pat. Nos. 4,683,202 and 4,683,195.

One Base Extension can also be carried out with Dark Reversible Terminator. In this format multiple replicative chips (or allele specific sequencing chips) are used. One extension is a single base addition of a mixture of dark nucleotides (a, c, g, and t) reversible terminator (for example, available from Foundation for Molecular Evolution). Each chip is different in one single base

In Four Base Extension format, multiple replicative chips (or allele specific sequencing chips) are used. One extension is a cycle of four nucleotides addition: a, c, g, or t. Three-cycle extension such as a, c, g, t; c, g, t, a; g, t, a, c, can be used to build longer distance when needed. The cycle can be optimized for specific target sequences. Each chip has incremental cycles, such as 0, 1, 2, 3, 4, 5, 6 cycles. Four labeled nucleotide (preferably different color/signal, but can be single color or fewer than four colors) addition as the detection cycle.

In some embodiment, four bases extension and detection are used as an example illustrated in FIGs. 1A, 1B, 2A and 2B. In this example, multiple replicate chips (e.g. allele specific sequencing chips or molecular clonal substrates) are used (numbered 1 to 5 for illustrative purpose). Capture probes with an identical sequence are attached to each chip and hybridize to the target molecules, which also have the same sequences. The target molecules can be obtained by amplification of a parent target molecule. One cycle (A, C, G, T) extension and detection is used. (FIG. 1A, FIG. 2A).

For each cycle, there will be a minimal of 1 base extension (extension 1) so each chip is different for at least one base. For example, if the template sequence is acgt, an ACGT cycle will only extend one base "T". Some cycles have more base extensions, for example, sequence of tgca will be extended by four bases. Because of repeating sequences such as tttggccaaaaa, more than four bases can be extended by one cycle. In this example, there will be 12 bases per cycle. To extend a 100 base fragment, we need about 20-50 cycles.

In one format, after each base addition (about 1 min), there is a washing step to remove the nucleotide and prepare for the next extension.

Alternatively, in another format, there is no washing between nucleotide extensions. Instead, apyrase is added to the extension buffer with DNA polymerase. Each nucleotide is added sequentially. By optimizing the extension mixture (amount of enzymes, concentrations of nucleotides, etc.), there will be sufficient amount of a particular amount of nucleotide when it is added to extend a base. Thereafter, the nucleotide will be degraded by apyrase. A new nucleotide is then added to start the next step of an extension cycle or the next extension cycle.

An example of Four Base Extension and Detection is depicted in FIG. 1B and FIG. 2B.

Preferably, unmodified nucleotides, as well as DNA polymerase with non or minimal modification, are used for the base extension. In general, unmodified nucleotides, as well as DNA polymerase with non or minimal modification, provides higher incorporation efficiency and fidelity that modified nucleotides and/or modified polymerase. The usage of unmodified nucleotides, as well as DNA polymerase with non or minimal modification also reduce the cost the sequencing.

In general, target nucleic acid templates are immobilized to solid support in any suitable format and the extension by polymerase such as DNA polymerase RNA polymerase, reverse transcriptase, is carried out in the presence of suitable buffer and nucleotides. In general, the extensions are carried out using suitable conditions known in the art.

### Base Detection

Detection bases can be labeled nucleotides such as dNTPs with labels.

In general, the base are detected by extending the stagger fragments by contacting the hybridization complexes sequentially with one of labeled dATP, dCTP, dGTP and dTTP, in the presence of a polymerase, and detecting the incorporation of the labeled dATP, dCTP, dGTP and dTTP to obtain a sequence read from each substrate.

In some embodiments, a mixture of labeled dATP, dCTP, dGTP and dTTP are used. Due to general low incorporation efficiency of the modified dNTPs, such as labeled dNTPs, only the first few bases are extend to generate strong signal. The possibility of "run-on" extension is rather low and the signal generated by such "run-on" extension can be filter out as noise using methods provided herein or known in the art.

In some embodiments, a mixture of labeled ddATP, ddCTP, ddGTP and ddTTP are used, and no "run-on" extension is permitted.

In general, only one round of interrogation that covers all four possible bases is carried for each staggered fragment. For example, sequential addition of one labeled dNTPs leads to on base a time (i.e. on each substrate). This generally results in short read (such as one base or a few bases) that could be assembled.

In some embodiments, a longer read is generated with more than one round of interrogation

In some embodiments, a mixture of labeled ddATP, ddCTP, ddGTP, ddTTP and small amount (<10% (e.g. 5, 6, 7, 8, or 9%) or <20% (e.g., 10, 11, 12, 13, 14, 15, 16, 17, 18, or 19%) of native dATP, dCTP, dGTP, and dTTP are added to provided controlled extension but prevent unnecessary "run-on".

In some embodiments, the labeled nucleotides such are reversible terminators, but they are not required. Multiple bases can be detected by the signal strength or in the case of reversible terminator, base addition detection.

Nucleotide reversible terminators are nucleotide analogues, which are modified with a reversible chemical moiety capping the 3'-OH group to temporarily terminate the polymerase reaction. In this way, only one nucleotide is incorporated into the growing DNA strand even in homopolymeric regions. For example, the 3' end can be capped with an amino-2-hydroxypropyl group. An allyl or a 2-nitrobenzyl group can also be used as the reversible moiety to cap the 3'-OH of the four nucleotides. Examples of reversible terminators include but are not limited to 3'-*O*-modified nucleotides such as 3'-O-allyl-dNTPs and 3'-O-(2-nitrobenzyl)-dNTPs. After detection of the cleavage site present on the solution probe, the 3'-OH of the primer extension products is regenerated through different deprotection methods. The capping moiety on the 3'-OH of the DNA extension product can be efficiently removed after detection of a cleavage site by a chemical method, enzymatic reaction or photolysis, i.e. the cap will be cleaved from the cleavage site. To sequence DNA, in some embodiments, templates containing homopolymeric regions are immobilized on Sepharose beads, and then extension-signal detection-deprotection cycles are conducted by using the nucleotide reversible terminators on the DNA beads to unambiguously decipher the sequence of DNA templates. In some embodiments, this reversible-terminator-sequencing approach is used in the subject methods to accurately determine DNA sequences. (The cap may be referred to herein as a "protective group").

Polynucleotide of the invention may be labeled. In some embodiments, a molecule or compound has at least one element, isotope or chemical compound attached to enable the detection of the compound. In general, labels of use in the invention include without limitation isotopic labels, which may be radioactive or heavy isotopes, magnetic labels, electrical labels, thermal labels, colored and luminescent dyes, enzymes and magnetic particles as well. Dyes of use in the invention may be chromophores, phosphors or fluorescent dyes, which due to their strong signals provide a good signal-to-noise ratio for decoding.

Many embodiments of the invention include the use of fluorescent labels. Suitable dyes for use in the invention include, but are not limited to, fluorescent lanthanide complexes, including those of Europium and Terbium, fluorescein, rhodamine, tetramethylrhodamine, eosin, erythrosin, coumarin, methyl-coumarins, pyrene, Malacite green, stilbene, Lucifer Yellow, Cascade Blue, Texas Red, and others described in the 6th Edition of the Molecular Probes Handbook by Richard P. Haugland. Commercially available fluorescent nucleotide analogues readily incorporated into the labeling oligonucleotides include, for example, Cy3-dCTP, Cy3-dUTP, Cy5-dCTP, Cy5- dUTP (Amersham Biosciences, Piscataway, New Jersey, USA), fluorescein-12-dUTP, tetramethylrhodamine-6-dUTP, Texas Red®-5-dUTP, Cascade Blue®-7-dUTP, BODIPY® FL-14-dUTP, BODIPY®R-14-dUTP, BODIPY® TR-14-dUTP, Rhodamine GreenTM-5-dUTP, Oregon Green® 488-5-dUTP, Texas Red®-12-dUTP, BODIPY® 630/650-14-dUTP, BODIPY® 650/665-1 4-dUTP, Alexa Fluor® 488-5-dUTP, Alexa Fluor® 532-5-dUTP, Alexa Fluor® 568-5-dUTP, Alexa Fluor® 594-5-dUTP, Alexa Fluor® 546-1 4-dUTP, fluorescein-12-UTP, tetramethylrhodamine-6-UTP, Texas Red®-5-UTP, Cascade Blue®-7-UTP, BODIPY® FL-14-UTP, BODIPY® TMR-14-UTP, BODIPY® TR-14-UTP, Rhodamine GreenTM-5-UTP, Alexa Fluor® 488-5-UTP, Alexa Fluor® 546-1 4-UTP (Molecular Probes, Inc. Eugene, OR, USA). Other fluorophores available for post-synthetic attachment include, inter alia, Alexa Fluor® 350, Alexa Fluor® 532, Alexa Fluor® 546, Alexa Fluor® 568, Alexa Fluor® 594, Alexa Fluor® 647, BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY 630/650, BODIPY 650/665, Cascade Blue, Cascade Yellow, Dansyl, lissamine rhodamine B, Marina Blue, Oregon Green 488, Oregon Green 514, Pacific Blue, rhodamine 6G, rhodamine green, rhodamine red, tetramethylrhodamine, Texas Red (available from Molecular Probes, Inc., Eugene, OR, USA), and Cy2, Cy3.5, Cy5.5, and Cy7 (Amersham Biosciences, Piscataway, NJ USA, and others).

A number of multiplex detection formats can be used, including either labeled/tagged bead sets (e.g., those produced by Luminex), in which each label is assigned to the individual probe-specific primer, or oligonucleotide arrays on slides, in which in which specific oligonucleotide spot/position is assigned to the individual probe-specific primer. The limited sequence complexity of the recovered target- specific probes provides conditions for easier and higher level multiplexing, especially using with universal and Zip-code/ID sequence tags. After the hybridization of the primers to the target-probe complex, the primers are extended by a nucleotide polymerase. Polymerase chain reaction is a technique well known in the relevant art. In certain embodiments, the polymerase is selected from an RNA polymerase and a reverse transcriptase.

Where an array is utilized, the detection phase of the process may involve scanning and identifying target polynucleotide sequences in the test sample. Scanning can be carried out by scanning probe microscopy (SPM) including scanning tunneling microscopy (STM) and atomic force microscopy (AFM), scanning electron microscopy, confocal microscopy, charge-coupled device, infrared microscopy, electrical conductance, and fluorescent or phosphor imaging, for example fluorescence resonance energy transfer (FRET). Optical interrogation/detection techniques include but are not limited to near-field scanning optical microscopy (NSOM), confocal microscopy and evanescent wave excitation. More specific versions of these techniques include far-field confocal microscopy, two-photon microscopy, wide-field epi-illumination, and total internal reflection (TIR) microscopy. Many of the above techniques can also be used in a spectroscopic mode. The actual detection means include charge coupled device (CCD) cameras and intensified CCDs, photodiodes and photomultiplier tubes. These means and techniques are well-known in the art. Various detection methods are disclosed in U.S. Patent Application Publication No. US 2004/0248144.

For multicolor imaging, signals of different wavelength can be obtained by multiple acquisitions or by simultaneous acquisition by splitting the signal, using RGB detectors or analyzing the whole spectrum (Richard Levenson, Cambridge Healthtech Institutes, Fifth Annual meeting on Advances in Assays, Molecular Labels, Signaling and Detection, May 17-18thWashington D.C.). Several spectral lines can acquired by the use of a filter wheel or a monochromater. Electronic tunable filters such as acoustic-optic tunable filters or liquid crystal tunable filters can be used to obtain multispectral imaging (e.g. Oleg Hait, Sergey Smirnov and Chieu D. Tran, 2001, Analytical Chemistry 73: 732-739). An alternative method to obtain a spectrum is hyperspectral imaging (Schultz et al., 2001, Cytometry 43:239-247).

Methods and apparatus for signal detection and processing of intensity data are disclosed in, for example, U.S. Pat. Nos. 5,143,854, 5,547,839, 5,578,832, 5,631,734, 5,800,992, 5,834,758; 5,856,092, 5,902,723, 5,936,324, 5,981,956, 6,025,601, 6,090,555, 6,141,096, 6,185,030, 6,201,639; 6,218,803; and 6,225,625, in U.S. Ser. No. 60/364,731 and in PCT Application PCT/US99/06097 (published as WO99/47964). Fluorescence imaging and software programs or algorithms for DNA sequence analysis and read interpretation are known to one of ordinary skill in the art and are disclosed in Harris TD, et al. "Single-Molecule DNA Sequencing of a Viral Genome" Science 4 April 2008: Vol. 320. no. 5872, pp. 106 - 109, which is herein incorporated by reference in its entirety. In some embodiments, Phred software is used for DNA sequence analysis. Phred reads DNA sequencer trace data, calls bases, assigns quality values to the bases, and writes the base calls and quality values to output files. Phred is a widely-used *program* for base calling DNA sequencing trace files. Phred can read trace data from SCF files and ABI model 373 and 377 DNA sequencer chromat files, automatically detecting the file format. After calling bases, phred writes the sequences to files in either FASTA format, the format suitable for XBAP, PHD format, or the SCF format. Quality values for the bases are written to FASTA format files or PHD files, which can be used by the phrap sequence assembly program in order to increase the accuracy of the assembled sequence. The quality value is a log-transformed error probability, specifically Q = -10 log₁₀( Pₑ) where Q and Pₑ are respectively the quality value and error probability of a particular base call. The phred quality values have been thoroughly tested for both accuracy and power to discriminate between correct and incorrect base-calls. Phred can use the quality values to perform sequence trimming.

In some embodiments, one detection cycle is performed by adding labeled A, C, G, T sequentially followed by washing and detecting after each addition. See FIG. 2B. In some embodiments, multiple detection cycles can be performed using nucleotides with removable labels.

### Processing of Raw Data and Analysis of Genetic Information with Computer Algorithm

Typically, identifying target polynucleotide sequence and integrating sequences to assemble genomic information is carried out with a computer. In some embodiments, the present invention also encompasses computer software or algorithm designed to analyze and assemble sequence information obtained via the methods of the present invention.

In terms of sequence read interpretation for the "in situ" arrays, reads at array features correspond to X-Y coordinates that map to the loci of interest. A "read" typically refers to an observed sequence derived from raw data, such as the order of detected signals corresponding to the cyclical addition of individual nucleotides. In some embodiments, the reads are checked against the expected reference genome sequence at the 10-bp loci for quality control. A reference sequence enables the use of short read length. Reads that have passed the quality control check are then combined to generate a consensus sequence at each locus. In one example, there are 10 unique probes per locus of interest minus any reads that have failed the quality control checks.

In terms of sequence read interpretation for the "lawn" approach, the reads are at random locations on a surface, e.g. a flow cell. In some embodiments, the reads are checked against the expected subset of reference genome sequence at the loci of interest for quality control. Reads that have passed the quality control check are mapped to the individual locus of interest. Reads corresponding to each locus are then combined to generate a consensus sequence. In one embodiment, there are more than 3,000 reads per 10-bp locus.

### Assembly of Sequence Reads

In another aspect, the present invention provides method for obtaining the sequence information of the target molecules by assembly the sequence read from each of the substrates. See FIG. 2B, for example. The sequence reads are obtained by base extension of a series of polynucleotide with different lengths due to the different base extension of the same capture probe using the same target molecules. As such, they represent continue fragments of the target molecule sequence and can be assembled to provide the continue sequence of the target molecule.

Computer program can be used to track the sequence reads obtained from the same capture probes on different substrates for the assembly.

### Multiple Targets

In some embodiments, multiple targets such as 10,000, 100,000, 1 million, 10 million, 100 million sequences or targets are sequenced simultaneous. Thus, for each substrates, there are a plurality of capture sites with each capture sites have different capture probes that recognize different targets. If the targets are fragments of a longer sequence, contigs can be assembly to obtain the longer sequence, such as the whole genome sequence.

### II. Substrate Format

In general, multiple target sequencing is typically done in chip format, but it can be performed in bead format as well.

By "chip" herein is meant a substrate having a suitable surface for the attachment of the target or probe. The geometric design of the chip an vary. For example, the chip can be a tube with the usable surface inside. Chips can be in flow cell format to facilitate liquid handling

In some embodiments, allele specific chips are used for sequencing. In some embodiments, capture probes are immobilized (or in situ synthesized) on chips. These probes can server as primer for sequencing. The same target are hybridized with a large number of chips, such as 5 (about 15 bases sequencing) to 40 chips (about 100 base sequencing) or 120 chips (about 300 base sequencing) or 300 chips (about 1,000 base sequencing)

In some embodiments, the chips are allele specific sequencing chips as disclosed in PCT/US2010/048526.

In some embodiments, the chip comprises random clusters started with single molecules (such as Illumina flow cells). The molecular clones of target molecules can be printed to many substrates to create replicate substrates for sequencing. In some embodiments, the chips are duplicating chips by nylon membrane impression and printing or other methods known in the art.

In some embodiments, the chip is a membrane multichip. Multilayered substrate with holes (1 micron to 50 micron) are generated. Target molecules are loaded into the holes with some holes with single molecule target. Targets are amplified within holes. The layers are peeled off. Each layer has some molecules attached to the holes. The layers are substantially similar in terms of molecules (copies of each other). These layers can be directly used or transferred to a suitable sequencing substrate for sequencing

Other chips can also be used in the present invention, include but are not limited to photo cleavable oligo multichip, multilayer substrates with holes, and nanopriting chip.

In some embodiments, the biotinylated beads is used to anchor the target sequence and the sequencing as carried out by performing the base incorporation in the bead system.

### III. Applications

The methods of the present invention provide several advantages. First, the sequencing method provided herein allow the use of unmodified nucleotide and enzymes which utility the natural DNA synthesis chemistry. This not only reduce the cost, but also increase the accuracy because the high-fidelity chemistry generated by the evolution process.

Furthermore, when loci- and allele-specific are used, they are SNP capable, and can carry multiple signal- reporting labels or ligands, provide for a higher level of multiplexing of diverse target sequences.

The present invention provides low-cost, high-throughput and accurate methods for sequencing target polynucleotide.

Moreover, the methods of the invention can be multiplexed to a very high degree. Samples can comprise pooled genomes of target and control subject populations respectively, since accurate analysis of allele frequencies can be accurately determined by single molecule counting. Since more than a single site on each molecule can be probed, haplotype information is easily determined. There is also the possibility of obtaining haplotype frequencies. Such methods are particularly applicable in association studies, where SNP frequencies are correlated with diseases in a population. The expense of single SNP typing reactions can be prohibitive when each study requires the performance of millions of individual reactions; the present invention permits millions of individual reactions to be performed and analyzed on a single array surface.

The methods of the present invention are useful in identifying high value polymorphisms located in regulatory elements and coding regions for a number of drug metabolizing enzyme and transporter (DMET) genes. Expression of these DMET genes will give information on the absorption, distribution, metabolism, and excretion profiles of a drug. Interpretation of complex transcriptional responses to various drugs and subsequent prediction of physiological effects is important for the development of effective therapeutics. The methods of the present invention can help draw links between gene expression profiles and physiological effects including a subjects' likely response to a drug candidate.

A wide variety of diseases can be detected by the process of the present invention, for example, infectious diseases caused by bacterial, viral, parasite, and fungal infectious agents. The resistance of various infectious agents to drugs can also be determined using the present invention.

Genetic diseases can also be detected by the process of the present invention. This can be carried out by prenatal or post-natal screening for chromosomal and genetic aberrations or for genetic diseases. Examples of detectable genetic diseases include: 21 hydroxylase deficiency, cystic fibrosis, Fragile X Syndrome, Turner Syndrome, Duchenne Muscular Dystrophy, Down Syndrome or other trisomies, heart disease, single gene diseases, HLA typing, phenylketonuria, sickle cell anemia, Tay-Sachs Disease, thalassemia, Klinefelter Syndrome, Huntington Disease, autoimmune diseases, lipidosis, obesity defects, hemophilia, inborn errors of metabolism, and diabetes.

Cancers which can be detected by the process of the present invention generally involve oncogenes, tumor suppressor genes, or genes involved in DNA amplification, replication, recombination, or repair. Examples of these include: BRCA1 gene, p53 gene, APC gene, Her2/Neu amplification, Bcr/Ab1, K-ras gene, and human papillomavirus Types 16 and 18. Various aspects of the present invention can be used to identify amplifications, large deletions as well as point mutations and small deletions/insertions of the above genes in the following common human cancers: leukemia, colon cancer, breast cancer, lung cancer, prostate cancer, brain tumors, central nervous system tumors, bladder tumors, melanomas, liver cancer, osteosarcoma and other bone cancers, testicular and ovarian carcinomas, head and neck tumors, and cervical neoplasms.

In the area of environmental monitoring, the present invention can be used for detection, identification, and monitoring of pathogenic and indigenous microorganisms in natural and engineered ecosystems and microcosms such as in municipal waste water purification systems and water reservoirs or in polluted areas undergoing bioremediation. It is also possible to detect plasmids containing genes that can metabolize xenobiotics, to monitor specific target microorganisms in population dynamic studies, or either to detect, identify, or monitor genetically modified microorganisms in the environment and in industrial plants.

The present invention can also be used in a variety of forensic areas, including for human identification for military personnel and criminal investigation, paternity testing and family relation analysis, HLA compatibility typing, and screening blood, sperm, or transplantation organs for contamination.

In the food and feed industry, the present invention has a wide variety of applications. For example, it can be used for identification and characterization of production organisms such as yeast for production of beer, wine, cheese, yogurt, bread, etc. Another area of use is with regard to quality control and certification of products and processes (e.g., livestock, pasteurization, and meat processing) for contaminants. Other uses include the characterization of plants, bulbs, and seeds for breeding purposes, identification of the presence of plant-specific pathogens, and detection and identification of veterinary infections.

In another aspect, the present disclosure provides a method for detecting microbial, such as by sequencing PCR products from virus/bacterial. The PCR Products can be hybridized with 5'-3' chips (direct sequencing) or 3'-5' chips (requires additional sequencing primer). Only 20-50 base sequencing is needed, about 10-20 chips. A chip density of 10k can produce approximately 200k to 500k base sequence.

In some embodiments, the sequence method provided herein is used to sequence pathogens/microbial genomes to identify species/strains.

For example, assuming a bacterial genome with a size of 5Mb (5X10⁶ bp), and each sequencing run reads 100bp. It is further assumed based on homopolymer calculation that there is on average 2.5 bp extension per cycle. Thus 40 cycles (100/2.5) are need, and each cycle needs 4 chips (one for each color of the label), which translates into total 160 chips for each sequencing run. Therefore, if there are 50x10³ spots (or capture sites) on each chip each with different capture probes, a total of 160 chips is needed for 1x sequencing of 5Mb (50x10³ x 100bp) = 5Mb.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### EXAMPLES

### Example 1

### Detecting Homopolymer

Sequencing of homopolymer (a stretch of G) was carried out with dark and labeled base mixture (1:9). If the labeled base incorporation = 50% for the mixture, the signal for 100% labeled G is 100% signal, then there was 50% signal for a single G, 75% signal for GG, and 87.5% signal for GGG.

## Claims

1. A method of sequencing a target nucleic acid, comprising:
a) providing a plurality of substrates, wherein said plurality of substrates is n, each substrate of said plurality of substrates is designed as a different substrate (i), wherein said (i) is an integer from 1 to n, wherein each different substrate of said plurality of substrates comprises a capture site comprising a capture probe, and wherein said capture probe comprises a sequence that is complementary to a portion of said target nucleic acid, wherein said target nucleic acid comprises a fragment of a source nucleic acid;
b) forming a hybridization complex on each of said different substrates (i), wherein said hybridization complex comprises said capture probe and said target nucleic acid, wherein said capture probe is hybridized to said portion of said target nucleic acid;
c) extending said capture probe in said hybridization complex on each of said different substrates (i) by repeating i-1 times the step of: contacting said hybridization complex on each of said different substrates (i) sequentially with 1) one of native dATP, dCTP, dGTP and dTTP; or 2) a mixture of two or three native dNTPs followed by one or more rounds of two or more different native dNTPs until all four native dNTPs are added at least once, in the presence of a polymerase, thereby extending said capture probe in said hybridization complex by one or more bases using said target nucleic acid in said hybridization complex as template, wherein said extending said capture probe in said hybridization complex occurs in a separate extension reaction for each of said different substrates (i);
d) further extending said capture probe in said hybridization complex on each of said different substrate (i) sequentially with one of labeled dATP, dCTP, dGTP and dTTP in the presence of a polymerase;
e) detecting incorporation of said labeled dATP, dCTP, dGTP and dTTP in said hybridization complex on each of said different substrates (i), thereby obtaining a sequence read from said hybridization complex on each of said different substrates (i); and
f) determining a nucleotide sequence of said target nucleic acid by assembling said sequence reads, wherein said assembling is performed on a computer.

2. The method of claim 1, wherein said n is an integer from 10 to 100.

3. The method of claim 1, wherein said n is 10.

4. The method of claim 1, wherein each of said different substrates (i) comprises a plurality of capture sites, wherein at least one of said capture sites on each of said different substrates comprises a capture probe of the same sequence.

5. The method of claim 1, wherein said capture probes are attached to a flat surface or a bead.

6. The method of claim 5, wherein said capture probes are synthesized or spotted on said flat surface.

7. The method of claim 5, wherein said flat surface is a flow cell.

8. The method of claim 5, wherein said capture probes are spotted at known locations on said flat surface.

9. The method of claim 1, wherein said portion of said target nucleic acid comprises a specific allele, locus of a particular genomic region, or an identifier sequence appended to said target nucleic acid.

10. The method of claim 1, wherein each of said plurality of substrates comprises a chip.

11. The method of claim 1, wherein said source nucleic acid is genomic DNA.

## Patentansprüche

1. Verfahren zur Sequenzierung einer Zielnukleinsäure, das Folgendes umfasst:
a) Bereitstellen einer Vielzahl von Substraten, wobei die Vielzahl von Substraten n ist, wobei jedes Substrat der Vielzahl von Substraten als verschiedenes Substrat (i) gebildet ist, wobei (i) eine ganze Zahl von 1 bis n ist, wobei jedes verschiedene Substrat der Vielzahl von Substraten eine Erfassungsstelle umfasst, die eine Erfassungssonde umfasst, und wobei die Erfassungssonde eine Sequenz umfasst, die komplementär zu einem Teil der Zielnukleinsäure ist, wobei die Zielnukleinsäure ein Fragment einer Quellennukleinsäure umfasst;
b) Bilden eines Hybridisierungskomplexes auf jedem der verschiedenen Substrate (i), wobei der Hybridisierungskomplex die Erfassungssonde und die Zielnukleinsäure umfasst, wobei die Erfassungssonde mit dem Teil der Zielnukleinsäure hybridisiert ist;
c) Erweitern der Erfassungssonde in dem Hybridisierungskomplex auf jedem der verschiedenen Substrate (i) durch i-1-faches Wiederholen der folgenden Schritte: Kontaktieren des Hybridisierungskomplexes auf jedem der verschiedenen Substrate (i) in Gegenwart einer Polymerase nacheinander mit 1) nativem dATP, dCTP, dGTP oder dTTP; oder 2) einer Mischung aus zwei oder drei nativen dNTPs, gefolgt von einer oder mehreren Runden von zwei oder mehr verschiedenen nativen dNTPs, bis alle vier nativen dNTPs mindestens einmal hinzugefügt werden, wodurch die Erfassungssonde in dem Hybridisierungskomplex durch eine oder mehrere Basen unter Verwendung der Zielnukleinsäure in dem Hybridisierungskomplex als Matrize erweitert wird, wobei das Erweitern der Erfassungssonde in dem Hybridisierungskomplex in einer separaten Erweiterungsreaktion für jedes der verschiedenen Substrate (i) erfolgt;
d) weiteres Erweitern der Erfassungssonde in dem Hybridisierungskomplex auf jedem der verschiedenen Substrate (i) nacheinander mit einem der markierten dATP, dCTP, dGTP und dTTP in Gegenwart einer Polymerase,
e) Erfassen der Eingliederung des markierten dATP, dCTP, dGTP und dTTP in dem Hybridisierungskomplex auf jedem der verschiedenen Substrate (i), wodurch eine Sequenz erhalten wird, die aus dem Hybridisierungskomplex auf jedem der verschiedenen Substrate (i) gelesen wird; und
f) Bestimmen einer Nukleotidsequenz der Zielnukleinsäure durch Zusammensetzen der Sequenzauslesungen, wobei das Zusammensetzen auf einem Computer durchgeführt wird.

2. Verfahren nach Anspruch 1, wobei n eine ganze Zahl von 10 bis 100 ist.

3. Verfahren nach Anspruch 1, wobei n 10 ist.

4. Verfahren nach Anspruch 1, wobei jedes der verschiedenen Substrate (i) eine Vielzahl von Erfassungsstellen umfasst, wobei mindestens eine der Erfassungsstellen auf jedem der verschiedenen Substrate eine Erfassungssonde der gleichen Sequenz umfasst.

5. Verfahren nach Anspruch 1, wobei die Erfassungssonden an einer flachen Oberfläche oder an einem Wulst befestigt sind.

6. Verfahren nach Anspruch 5, wobei die Erfassungssonden auf der flachen Oberfläche synthetisiert oder gefleckt werden.

7. Verfahren nach Anspruch 5, wobei die flache Oberfläche eine Durchflusszelle ist.

8. Verfahren nach Anspruch 5, wobei die Erfassungssonden an bekannten Stellen auf der flachen Oberfläche gefleckt werden.

9. Verfahren nach Anspruch 1, wobei der Teil der Zielnukleinsäure ein spezifisches Allel, einen Locus einer bestimmten genomischen Region oder eine Kennungssequenz, die an die Zielnukleinsäure angefügt ist, umfasst.

10. Verfahren nach Anspruch 1, wobei jedes der Vielzahl von Substraten einen Chip umfasst.

11. Verfahren nach Anspruch 1, wobei die Quellennukleinsäure genomische DNA ist.

## Revendications

1. Procédé de séquençage d'un acide nucléique cible, comprenant :
a) la mise à disposition d'une pluralité de substrats, ladite pluralité de substrats étant représentée par n, chaque substrat de ladite pluralité de substrats se présentant sous la forme d'un substrat (i) différent, (i) représentant un nombre entier de 1 à n, chaque substrat différent de ladite pluralité de substrats comprenant un site de capture comprenant une sonde de capture, et ladite sonde de capture comprenant une séquence qui est complémentaire d'une partie dudit acide nucléique cible, ledit acide nucléique cible comprenant un fragment d'un acide nucléique source ;
b) la formation d'un complexe d'hybridation sur chacun desdits différents substrats (i), ledit complexe d'hybridation comprenant ladite sonde de capture et ledit acide nucléique cible, ladite sonde de capture étant hybridée à ladite partie dudit acide nucléique cible ;
c) l'extension de ladite sonde de capture dans ledit complexe d'hybridation sur chacun desdits différents substrats (i) en répétant i-1 fois l'étape consistant à : mettre en contact ledit complexe d'hybridation sur chacun desdits différents substrats (i) de manière séquentielle avec 1) un des éléments natifs suivants : dATP, dCTP, dGTP et dTTP ; ou 2) un mélange de deux ou trois dNTP natifs suivis d'au moins un cycle d'au moins deux dNTP natifs différents jusqu'à ce que les quatre dNTP natifs soient ajoutés au moins une fois, en présence d'une polymérase, et ainsi étendre ladite sonde de capture dans ledit complexe d'hybridation d'au moins une base à l'aide dudit acide nucléique cible dans ledit complexe d'hybridation comme modèle, ladite extension de ladite sonde de capture dans ledit complexe d'hybridation se produisant dans une réaction d'extension séparée pour chacun desdits différents substrats (i) ;
d) l'extension supplémentaire de ladite sonde de capture dans ledit complexe d'hybridation sur chacun desdits différents substrats (i) de manière séquentielle avec un des éléments marqués suivants : dATP, dCTP, dGTP et dTTP en présence d'une polymérase ;
e) la détection de l'incorporation desdits dATP, dCTP, dGTP et dTTP marqués dans ledit complexe d'hybridation sur chacun desdits différents substrats (i), permettant ainsi d'obtenir une séquence lue à partir dudit complexe d'hybridation sur chacun desdits différents substrats (i) ; et
f) la détermination d'une séquence nucléotidique dudit acide nucléique cible par l'assemblage desdites lectures de séquences, ledit assemblage étant réalisé sur un ordinateur.

2. Procédé selon la revendication 1, dans lequel ledit n est un nombre entier de 10 à 100.

3. Procédé selon la revendication 1, dans lequel ledit n est 10.

4. Procédé selon la revendication 1, dans lequel chacun desdits différents substrats (i) comprend une pluralité de sites de capture, au moins un desdits sites de capture sur chacun desdits différents substrats comprenant une sonde de capture de la même séquence.

5. Procédé selon la revendication 1, dans lequel lesdites sondes de capture sont fixées à une surface plate ou une bille.

6. Procédé selon la revendication 5, dans lequel lesdites sondes de capture sont synthétisées ou repérées sur ladite surface plate.

7. Procédé selon la revendication 5, dans lequel ladite surface plate est une cellule de mesure.

8. Procédé selon la revendication 5, dans lequel lesdites sondes de capture sont repérées à des emplacements connus sur ladite surface plate.

9. Procédé selon la revendication 1, dans lequel ladite partie dudit acide nucléique cible comprend un allèle spécifique, le locus d'une région génomique particulière ou une séquence d'identifiants jointe audit acide nucléique cible.

10. Procédé selon la revendication 1, dans lequel chaque substrat de ladite pluralité de substrats comprend une puce.

11. Procédé selon la revendication 1, dans lequel ledit acide nucléique source est de l'ADN génomique.
